# EUROPEAN PATENT APPLICATION

(11) **EP 1 671 972 A1**
(43) Date of publication of application: **21.06.2006**
(21) Application number: 04106044.3
(22) Date of filing: 24.11.2004
(51) Int. Cl.: C07D 487/04, A61K 31/407, A61K 31/4184, A61P 11/06, A61P 37/08

(54) **Octahydropyrrolo[3,4-c]pyrrole derivatives**

(71) Applicant: Pfizer Limited, Sandwich, Kent CT13 9NJ (GB)
(72) Inventor: LANE, Charlotte Pfizer Limited, Sandwich, Kent CT13, 9NJ (GB)
(74) Representative: Bridle, Andrew Barry

(57) **Abstract**

The present invention relates to octahydropyrrolo[3,4-c]pyrrole derivatives of formula (I): 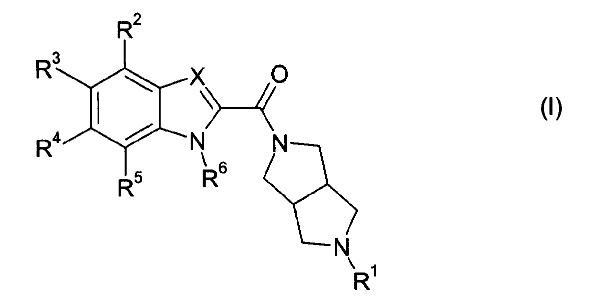 and to processes for the preparation thereof, compositions containing the same and the uses thereof.

## Description

This invention relates to octahydropyrrolo[3,4-c]pyrrole derivatives and to processes for the preparation of, compositions containing and the uses of, such derivatives.

The octahydropyrrolo[3,4-c]pyrrole derivatives of the present invention are histamine H₄ receptor ligands and have therefore a number of therapeutic applications, particularly in the treatment of asthma and allergic rhinitis.

The histamine H₄ receptor has recently been identified. It is a 390 aminoacid, seven-transmembrane G protein coupled receptor with approximately 40 % homology to the histamine H₃ receptor. In contrast to the H₃ receptor which is primarily located in the brain, the H₄ receptor is expressed at greater levels in eosinophils and mast cells, among other inflammatory cells.

H₄ receptor ligands should thus be suitable for the treatment of various inflammatory disorders. Examples of diseases where treatment with H₄ ligands is particularly appropriate are inflammatory bowel disease, Crohn's disease, colitis ulcerosa, dermatitis, psoriasis, conjunctivitis, rheumatoid arthritis, respiratory diseases such as adult respiratory distress syndrome, acute respiratory distress syndrome, bronchitis, chronic bronchitis, chronic obstructive pulmonary disease, cystic fibrosis, asthma, emphysema, rhinitis, chronic sinusitis, allergy, allergy-induced airway responses, allergic rhinitis, viral rhinitis, non-allergic rhinitis, perennial and seasonal rhinitis, nasal congestion and allergic congestion.

Recently some histamine H₄ receptor ligands have been developed. An overview of the current advance in H₄ ligand research and patenting is given in *Expert Opin. Ther. Patents* (2003) 13(6). Examples of Histamine H₄ receptor ligands can be found in WO 02/07548, WO 04/022537 and in Terzioglu et al., *J. Bioorg. Med. Chem. Lett. 14 (2004),* 5251-5256.

Although H₄ ligands are known there is still a need to further provide new H₄ ligands that are good drug candidates. In particular, preferred compounds should bind potently to the histamine H₄ receptor whilst showing little affinity for other receptors. They should be well absorbed from the gastrointestinal tract, be metabolically stable and possess favourable pharmacokinetic properties. They should be non-toxic and demonstrate few side-effects.

The present invention thus relates to octahydropyrrolo[3,4-c]pyrrole derivatives of formula (I): 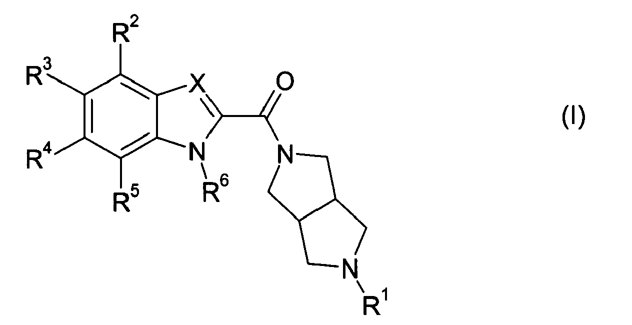 or pharmaceutically acceptable salts and solvates thereof, wherein:
- R¹ is H or (C₁-C₄) alkyl optionally substituted with a hydroxy;
- X is N or C-R⁹ wherein R⁹ is H or methyl;
- R⁶ is H or methyl; and
- R², R³, R⁴ and R⁵ are independently selected from H, halo, cyano, (C₁-C₄)alkyl, (C₁-C₄)alkoxy, trifluoromethyl, trifluoromethoxy, hydroxy, (CH₂)ₙ-C(O)O-R⁷, (CH₂)ₙ-O-(CH₂)ₘ-R⁸ and (CH₂)ₙ-R⁸,
wherein n and m are both independently 0 or 1, R⁷ is H or (C₁₋C₄)alkyl and R⁸ is phenyl.

It has been demonstrated that these compounds are ligands of the Histamine H₄ receptor.

In the here above formula "halo" denotes a halogen atom selected from the group consisting of fluoro, chloro, bromo and iodo, in particular fluoro or chloro.

(C₁-C₄)alkyl radicals denote a straight-chain or branched group containing 1, 2, 3 or 4 carbon atoms. This also applies if they carry substituents such as a hydroxy substitutent or occur as substituents of other radicals, for example in (C₁-C₄)alkoxy radicals Examples of suitable (C₁-C₄)alkyl radicals are methyl, ethyl, *n*-propyl, *iso*-propyl, *n*-butyl, *iso*-butyl, *sec*-butyl and *tert*-butyl. Examples of suitable (C₁-C₄)alkoxy radicals are methoxy, ethoxy, n-propyloxy, *iso*-propyloxy, *n*-butyloxy, *iso*-butyloxy, *sec*-butyloxy and *tert-*butyloxy. Examples of suitable (C₁-C₄)alkyl radicals substituted by a hydroxy radical are hydroxymethyl, 1-hydroxyethyl, 2-hydroxyethyl, 1-hydroxypropyl, 2-hydroxypropyl, 3-hydroxypropyl etc...

According to a first aspect, the compounds of formula (I) wherein R¹ is H or methyl are preferred. Preferably, R¹ is methyl.

According to another aspect, the compounds of formula (I) wherein R², R³, R⁴ and R⁵ are independently selected from H, halo, methyl, hydroxy, phenyl, and COOH are preferred. Preferably, halo is selected from fluoro, chloro and bromo. More preferably, R², R³, R⁴ and R⁵ are independently selected from H, fluoro, chloro, bromo, hydroxy and methyl.

According to another aspect, the compounds of formula (I) wherein R⁶ is H are preferred.

According to another aspect, the compounds of formula (I) wherein X is N or CH are preferred.

According to a further aspect, the compounds of formula (I) wherein R¹ is H or methyl and R², R³, R⁴ and R⁵ are independently selected from H, halo, methyl, hydroxy, phenyl, and COOH.

According to a further aspect, the compounds of formula (I) wherein R¹ is methyl, R⁶ is H, X is N or CH and R², R³, R⁴ and R⁵ are independently selected from H, fluoro, chloro, bromo, hydroxy and methyl.

According to a further aspect, the compounds of formula (I) wherein at least two of R², R³, R⁴ and R⁵ are H are preferred.

According to another preferred aspect, the compounds of formula (I) have the Cis configuration, i.e. the (3a*R*,6a*S*) configuration as follows: 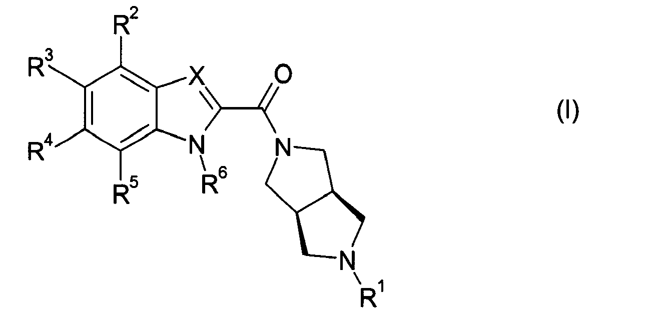 wherein R¹, R², R³, R⁴, R⁵, R⁶ and X are as defined above.

Pharmaceutically acceptable salts of the compounds of formula (I) include the acid addition and base salts thereof.

Suitable acid addition salts are formed from acids which form non-toxic salts. Examples include the acetate, aspartate, benzoate, besylate, bicarbonate/carbonate, bisulphate/sulphate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, saccharate, stearate, succinate, tartrate, tosylate and trifluoroacetate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts.

Hemisalts of acids and bases may also be formed, for example, hemisulphate and hemicalcium salts.

For a review on suitable salts, see Handbook of Pharmaceutical Salts: Properties, Selection, and Use by Stahl and Wermuth (Wiley-VCH, Weinheim, Germany, 2002).

Pharmaceutically acceptable salts of the compounds of formula (I) may be prepared by one or more of three methods:
(i) by reacting the compound of formula (I) with the desired acid or base;
(ii) by removing an acid- or base-labile protecting group from a suitable precursor of the compound of formula (I) or by ring-opening a suitable cyclic precursor, for example, a lactone or lactam, using the desired acid or base; or
(iii) by converting one salt of the compound of formula (I) to another by reaction with an appropriate acid or base or by means of a suitable ion exchange column.

All three reactions are typically carried out in solution. The resulting salt may precipitate out and be collected by filtration or may be recovered by evaporation of the solvent. The degree of ionisation in the resulting salt may vary from completely ionised to almost non-ionised.

The compounds of the invention may exist in both unsolvated and solvated forms. The term 'solvate' is used herein to describe a molecular complex comprising the compound of the invention and a stoichiometric amount of one or more pharmaceutically acceptable solvent molecules, for example, ethanol. The term 'hydrate' is employed when said solvent is water.

Included within the scope of the invention are complexes such as clathrates, drug-host inclusion complexes wherein, in contrast to the aforementioned solvates, the drug and host are present in stoichiometric or non-stoichiometric amounts. Also included are complexes of the drug containing two or more organic and/or inorganic components which may be in stoichiometric or non-stoichiometric amounts. The resulting complexes may be ionised, partially ionised, or non-ionised. For a review of such complexes, see J Pharm Sci, 64 (8), 1269-1288, by Haleblian (August 1975).

Hereinafter all references to compounds of formula (I) include references to salts, solvates and complexes thereof and to solvates and complexes of salts thereof.

The compounds of the invention include compounds of formula (I) as hereinbefore defined, including all polymorphs and crystal habits thereof, prodrugs and isomers thereof (including optical, geometric and tautomeric isomers) as hereinafter defined and isotopically-labeled compounds of formula (I).

As indicated, so-called 'pro-drugs' of the compounds of formula (I) are also within the scope of the invention. Thus certain derivatives of compounds of formula (I) which may have little or no pharmacological activity themselves can, when administered into or onto the body, be converted into compounds of formula (I) having the desired activity, for example, by hydrolytic cleavage. Such derivatives are referred to as 'prodrugs'. Further information on the use of prodrugs may be found in Pro-drugs as Novel Delivery Systems, Vol. 14, ACS Symposium Series (T. Higuchi and W. Stella) and Bioreversible Carriers in Drug Design, Pergamon Press, 1987 (ed. E. B. Roche, American Pharmaceutical Association).

Prodrugs in accordance with the invention can, for example, be produced by replacing appropriate functionalities present in the compounds of formula (I) with certain moieties known to those skilled in the art as 'pro-moieties' as described, for example, in Design of Prodrugs by H. Bundgaard (Elsevier, 1985).

Some examples of prodrugs in accordance with the invention include:
(i) where the compound of formula (I) contains a carboxylic acid functionality (-COOH), an ester thereof, for example, a compound wherein the hydrogen of the carboxylic acid functionality of the compound of formula (I) is replaced by (C₁-C₈)alkyl;
(ii) where the compound of formula (I) contains an alcohol functionality (-OH), an ether thereof, for example, a compound wherein the hydrogen of the alcohol functionality of the compound of formula (I) is replaced by (C₁-C₆)alkanoyloxymethyl; and
(iii) where the compound of formula (I) contains a primary or secondary amino functionality (-NH₂ or -NHR where R is not H), an amide thereof, for example, a compound wherein, as the case may be, one or both hydrogens of the amino functionality of the compound of formula (I) is/are replaced by (C₁-C₁₀)alkanoyl.

Further examples of replacement groups in accordance with the foregoing examples and examples of other prodrug types may be found in the aforementioned references.

Moreover, certain compounds of formula (I) may themselves act as prodrugs of other compounds of formula (I).

Also included within the scope of the invention are metabolites of compounds of formula (I), that is, compounds formed *in vivo* upon administration of the drug. Some examples of metabolites in accordance with the invention include:
(i) where the compound of formula (I) contains a methyl group, an hydroxymethyl derivative thereof (-CH₃ → -CH₂OH):
(ii) where the compound of formula (I) contains an alkoxy group, an hydroxy derivative thereof (-OR → -OH);
(iii) where the compound of formula (I) contains a tertiary amino group, a secondary amino derivative thereof (-NR^{a}R^{b} → -NHR^{a} or -NHR^{b});
(iv) where the compound of formula (I) contains a secondary amino group, a primary derivative thereof (-NHR^{a} → -NH₂);
(v) where the compound of formula (I) contains a phenyl moiety, a phenol derivative thereof (-Ph → -PhOH); and
(vi) where the compound of formula (I) contains an amide group, a carboxylic acid derivative thereof (-CONR^{c}R^{d} → COOH).

Compounds of formula (I) containing one or more asymmetric carbon atoms can exist as two or more stereoisomers. Where structural isomers are interconvertible via a low energy barrier, tautomeric isomerism ('tautomerism') can occur. This can take the form of proton tautomerism in compounds of formula (I) containing, for example, an imino, keto, or oxime group, or so-called valence tautomerism in compounds which contain an aromatic moiety. It follows that a single compound may exhibit more than one type of isomerism.

Included within the scope of the present invention are all stereoisomers, geometric isomers and tautomeric forms of the compounds of formula (I), including compounds exhibiting more than one type of isomerism, and mixtures of one or more thereof. Also included are acid addition or base salts wherein the counterion is optically active, for example, *d*-lactate or *l-*lysine, or racemic, for example, *dl*-tartrate or *dl*-arginine.

Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC).

Alternatively, the racemate (or a racemic precursor) may be reacted with a suitable optically active compound, for example, an alcohol, or, in the case where the compound of formula (I) contains an acidic or basic moiety, a base or acid such as 1-phenylethylamine or tartaric acid. The resulting diastereomeric mixture may be separated by chromatography and/or fractional crystallization and one or both of the diastereoisomers converted to the corresponding pure enantiomer(s) by means well known to a skilled person.

Chiral compounds of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% by volume of isopropanol, typically from 2% to 20%, and from 0 to 5% by volume of an alkylamine, typically 0.1% diethylamine. Concentration of the eluate affords the enriched mixture.

Stereoisomeric conglomerates may be separated by conventional techniques known to those skilled in the art - see, for example, Stereochemistry of Organic Compounds by E. L. Eliel and S. H. Wilen (Wiley, New York, 1994).

The present invention includes all pharmaceutically acceptable isotopically-labelled compounds of formula (I) wherein one or more atoms are replaced by atoms having the same atomic number, but an atomic mass or mass number different from the atomic mass or mass number which predominates in nature.

Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S.

Certain isotopically labelled compounds of formula (I), for example, those incorporating a radioactive isotope, are useful in drug and/or substrate tissue distribution studies. The radioactive isotopes tritium, *i.e.* ³H, and carbon-14, *i.e.* ¹⁴C, are particularly useful for this purpose in view of their ease of incorporation and ready means of detection.

Substitution with heavier isotopes such as deuterium, *i.e.* ²H, may afford certain therapeutic advantages resulting from greater metabolic stability, for example, increased *in vivo* half-life or reduced dosage requirements, and hence may be preferred in some circumstances.

Substitution with positron emitting isotopes, such as ¹¹C, ¹⁸F, ¹⁵O and ¹³N, can be useful in Positron Emission Topography (PET) studies for examining substrate receptor occupancy.

Isotopically labelled compounds of formula (I) can generally be prepared by conventional techniques known to those skilled in the art or by processes analogous to those described in the accompanying Examples and Preparations using an appropriate isotopically labelled reagent in place of the non-labelled reagent previously employed.

Pharmaceutically acceptable solvates in accordance with the invention include those wherein the solvent of crystallization may be isotopically substituted, e.g. D₂O, d₆-acetone, d₆-DMSO.

The Compounds of the general formula (I) according to the present invention may be prepared using conventional procedures such as by the following illustrative methods in which R¹, R², R³, R⁴, R⁵, R⁶ and X are as previously defined unless otherwise stated.

The compound of formula (I) may be prepared by coupling an acid of formula (II) : 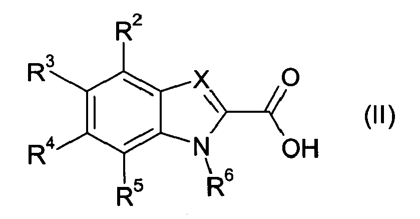 with an amine of formula (III): 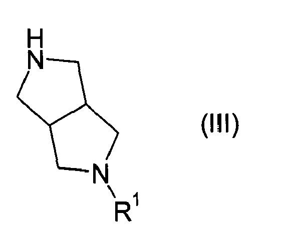 wherein R¹, R², R³, R⁴, R⁵, R⁶ and X are as previously defined.

The coupling of the acid (II) to the amine (III) is generally carried out in an excess of said amine, with a conventional coupling agent (e.g. 1-(3-dimethylaminopropyl)-3-ethylcarbodiimide hydrochloride, O-benzotriazo-1-yl-*N,N,N'N'*-tetramethyluronium hexafluorophosphate, *N,N'-*dicyclohexylcarbodiimide), optionally in the presence of a catalyst (e.g. 1-hydroxybenzotriazole hydrate or 1-hydroxy-7-azabenzotriazole), and optionally in the presence of a tertiary amine base (e.g. *N-*methylmorpholine, triethylamine or *N,N*-diisopropylethylamine). The reaction may be undertaken in a suitable solvent such as pyridine, N,N-dimethylformamide, tetrahydrofuran, dimethylsulfoxide, dichloromethane or ethyl acetate, and at temperature comprised between 10°C and 40°C (room temperature).
Preferably, the coupling is carried out using an excess of the amine (1.2-1.5 equivalents), with O-benzotriazo-1-yl-*N,N,N'N*'-tetramethyluronium hexafluorophosphate, *N,N*'-dicyclohexylcarbodiimide as coupling agent. The reaction is also preferably undertaken using N,N-dimethylformamide as solvent.

The amine of formula (III) may be prepared as described in the literature (J. *Heterocyclic. Chem* 1983, ***20*,** 321).

The acids of formula (II) where X is C-R⁹, wherein R⁹ is as previously defined, are either commercially available or may be prepared according to well-known methods described in the literature.

The acids of formula (II) where X is N may be prepared by oxidation of an alcohol of formula (IV) : 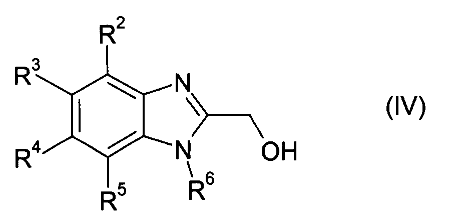 wherein R², R³, R⁴, R⁵ and R⁶ are as previously defined.

The oxidation is generally carried out using a conventional oxidising agent (e.g. potassium permanganate) optionally in the presence of a base (e.g. NaOH) in a suitable solvent (e.g. water) at a temperature comprised between 40°C and 100°C.
Preferably the oxidation is carried out using potassium permanganate in the presence of sodium hydroxide using water as solvent with a reaction temperature of 100°C for 1 hour and 30 minutes.

The alcohol of formula (IV) may be prepared by condensation of an aniline of formula (V) with glycolic acid : 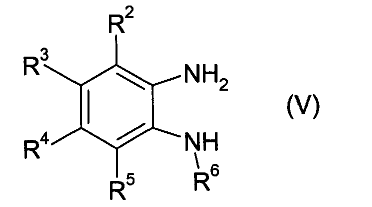 wherein R², R³, R⁴, R⁵ and R⁶ are as previously defined.

The condensation is generally carried out in the presence of an acid catalyst (e.g. HCl) in a suitable solvent (e.g. water) at a temperature comprised between 40°C and 100°C.
Preferably the condensation is carried out using 6N aqueous hydrochloric acid as solvent at a temperature of 100°C with a reaction time of 16 hours.

The aniline of formula (V) is either commercially available or may be prepared by conventional methods well known to the one skilled in the art (e.g. nitration, reduction, alkylation, chlorination) from commercially available material as detailed in the experimental section.

Alternatively the compound of formula (I) where X is N may be prepared by coupling a trichloromethylbenzimidazole of formula (VI): 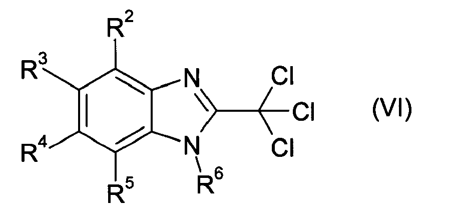 with an amine of formula (III) : 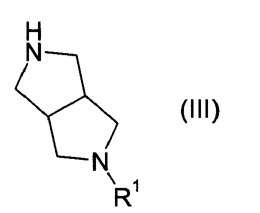 wherein R¹, R², R³, R⁴, R⁵ and R⁶ are as previously defined.

The coupling is generally carried out with an excess of the amine (III), optionally in the presence of a base (e.g. triethylamine, N,N-diethylisopropylamine, potassium carbonate) in a suitable aqueous solvent or mixture of solvents (e.g. acetonitrile, tetrahydrofuran, water) at a temperature comprised between 10°C and 40°C (room temperature).

The compound of formula (VI) may be prepared by condensation of an aniline of formula (V): 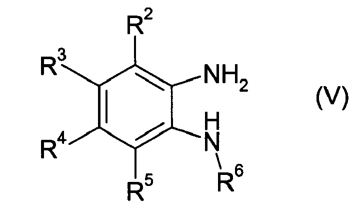 with a trichloromethylacetimidate ester of formula (VII): 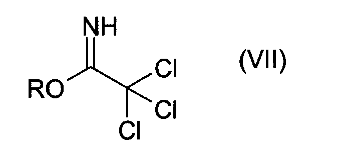 wherein R is methyl or ethyl and R², R³, R⁴, R⁵ and R⁶ are as previously defined.

The coupling is generally carried out with an excess of the acetimidate in a suitable solvent (e.g. AcOH) at a temperature comprised between 10°C and 40°C (room temperature).

The aniline of formula (V) is prepared as described previously and the trichloromethylacetimidate of formula (VII) is commercially available.

All of the above reactions and the preparations of novel starting materials using in the preceding methods are conventional and appropriate reagents and reaction conditions for their performance or preparation as well as procedures for isolating the desired products will be well-known to those skilled in the art with reference to literature precedents and the examples and preparations hereto.

The compounds of the invention intended for pharmaceutical use may be administered as crystalline or amorphous products. They may be obtained, for example, as solid plugs, powders, or films by methods such as precipitation, crystallization, freeze drying, spray drying, or evaporative drying. Microwave or radio frequency drying may be used for this purpose.

They may be administered alone or in combination with one or more other compounds of the invention or in combination with one or more other drugs (or as any combination thereof). Generally, they will be administered as a formulation in association with one or more pharmaceutically acceptable excipients. The term 'excipient' is used herein to describe any ingredient other than the compound(s) of the invention. The choice of excipient will to a large extent depend on factors such as the particular mode of administration, the effect of the excipient on solubility and stability, and the nature of the dosage form.

Pharmaceutical compositions suitable for the delivery of compounds of the present invention and methods for their preparation will be readily apparent to those skilled in the art. Such compositions and methods for their preparation may be found, for example, in Remington's Pharmaceutical Sciences, 19th Edition (Mack Publishing Company, 1995).

The compounds of the invention may be administered orally. Oral administration may involve swallowing, so that the compound enters the gastrointestinal tract, or buccal or sublingual administration may be employed by which the compound enters the blood stream directly from the mouth.

Formulations suitable for oral administration include solid formulations such as tablets, capsules containing particulates, liquids, or powders, lozenges (including
liquid-filled), chews, multi- and nano-particulates, gels, solid solution, liposome, films, ovules, sprays and liquid formulations.

Liquid formulations include suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The compounds of the invention may also be used in fast-dissolving, fast-disintegrating dosage forms such as those described in Expert Opinion in Therapeutic Patents, 11 (6), 981-986, by Liang and Chen (2001).

For tablet dosage forms, depending on dose, the drug may make up from 1 weight % to 80 weight % of the dosage form, more typically from 5 weight % to 60 weight % of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl-substituted hydroxypropyl cellulose, starch, pregelatinised starch and sodium alginate. Generally, the disintegrant will comprise from 1 weight % to 25 weight %, preferably from 5 weight % to 20 weight % of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally comprise surface active agents, such as sodium lauryl sulfate and polysorbate 80, and glidants such as silicon dioxide and talc. When present, surface active agents may comprise from 0.2 weight % to 5 weight % of the tablet, and glidants may comprise from 0.2 weight % to 1 weight % of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 weight % to 10 weight %, preferably from 0.5 weight % to 3 weight % of the tablet.

Other possible ingredients include anti-oxidants, colorants, flavouring agents, preservatives and taste-masking agents.

Exemplary tablets contain up to about 80% drug, from about 10 weight % to about 90 weight % binder, from about 0 weight % to about 85 weight % diluent, from about 2 weight % to about 10 weight % disintegrant, and from about 0.25 weight % to about 10 weight % lubricant.

Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated.

The formulation of tablets is discussed in Pharmaceutical Dosage Forms: Tablets, Vol. 1, by H. Lieberman and L. Lachman (Marcel Dekker, New York, 1980).

Consumable oral films for human or veterinary use are typically pliable water-soluble or water-swellable thin film dosage forms which may be rapidly dissolving or mucoadhesive and typically comprise a compound of formula (I), a film-forming polymer, a binder, a solvent, a humectant, a plasticiser, a stabiliser or emulsifier, a viscosity-modifying agent and a solvent. Some components of the formulation may perform more than one function.

The compound of formula (I) may be water-soluble or insoluble. A water-soluble compound typically comprises from 1 weight % to 80 weight %, more typically from 20 weight % to 50 weight %, of the solutes. Less soluble compounds may comprise a greater proportion of the composition, typically up to 88 weight % of the solutes. Alternatively, the compound of formula (I) may be in the form of multiparticulate beads.

The film-forming polymer may be selected from natural polysaccharides, proteins, or synthetic hydrocolloids and is typically present in the range 0.01 to 99 weight %, more typically in the range 30 to 80 weight %.

Other possible ingredients include anti-oxidants, colorants, flavourings and flavour enhancers, preservatives, salivary stimulating agents, cooling agents, co-solvents (including oils), emollients, bulking agents, anti-foaming agents, surfactants and taste-masking agents.

Films in accordance with the invention are typically prepared by evaporative drying of thin aqueous films coated onto a peelable backing support or paper. This may be done in a drying oven or tunnel, typically a combined coater dryer, or by freeze-drying or vacuuming.

Solid formulations for oral administration may be formulated to be immediate and/or modified release. Modified release formulations include delayed-, sustained-, pulsed-, controlled-, targeted and programmed release.

Suitable modified release formulations for the purposes of the invention are described in US Patent No. 6,106,864. Details of other suitable release technologies such as high energy dispersions and osmotic and coated particles are to be found in Pharmaceutical Technology On-line, 25(2), 1-14, by Verma *et al* (2001). The use of chewing gum to achieve controlled release is described in WO 00/35298.

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

The solubility of compounds of formula (I) used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and poly(*dl*-lactic-coglycolic)acid (PGLA) microspheres.

The compounds of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, J Pharm Sci, 88 (10), 955-958, by Finnin and Morgan (October 1999).

Other means of topical administration include delivery by electroporation, iontophoresis, phonophoresis, sonophoresis and microneedle or needle-free (e.g. Powderject™, Bioject™, etc.) injection.

Formulations for topical administration may be formulated to be immediate and/or modified release. Modified release formulations include delayed-, sustained-, pulsed-, controlled-, targeted and programmed release.

The compounds of the invention can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurised container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane. For intranasal use, the powder may comprise a bioadhesive agent, for example, chitosan or cyclodextrin.

The pressurised container, pump, spray, atomizer, or nebuliser contains a solution or suspension of the compound(s) of the invention comprising, for example, ethanol, aqueous ethanol, or a suitable alternative agent for dispersing, solubilising, or extending release of the active, a propellant(s) as solvent and an optional surfactant, such as sorbitan trioleate, oleic acid, or an oligolactic acid.

Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

Capsules (made, for example, from gelatin or hydroxypropylmethylcellulose), blisters and cartridges for use in an inhaler or insufflator may be formulated to contain a powder mix of the compound of the invention, a suitable powder base such as lactose or starch and a performance modifier such as *I*-leucine, mannitol, or magnesium stearate. The lactose may be anhydrous or in the form of the monohydrate, preferably the latter. Other suitable excipients include dextran, glucose, maltose, sorbitol, xylitol, fructose, sucrose and trehalose.

A suitable solution formulation for use in an atomiser using electrohydrodynamics to produce a fine mist may contain from 1µg to 20mg of the compound of the invention per actuation and the actuation volume may vary from 1µl to 100µl. A typical formulation may comprise a compound of formula (I), propylene glycol, sterile water, ethanol and sodium chloride. Alternative solvents which may be used instead of propylene glycol include glycerol and polyethylene glycol.

Suitable flavours, such as menthol and levomenthol, or sweeteners, such as saccharin or saccharin sodium, may be added to those formulations of the invention intended for inhaled/intranasal administration.

Formulations for inhaled/intranasal administration may be formulated to be immediate and/or modified release using, for example, PGLA. Modified release formulations include delayed-, sustained-, pulsed-, controlled-, targeted and programmed release.

In the case of dry powder inhalers and aerosols, the dosage unit is determined by means of a valve, which delivers a metered amount. Units in accordance with the invention are typically arranged to administer a metered dose or "puff" containing from 1 µg to 4000 µg of the compound of formula (I). The overall daily dose will typically be in the range 1 µg to 20 mg which may be administered in a single dose or, more usually, as divided doses throughout the day.
The compounds of the invention may be administered rectally or vaginally, for example, in the form of a suppository, pessary, or enema. Cocoa butter is a traditional suppository base, but various alternatives may be used as appropriate.

Formulations for rectal/vaginal administration may be formulated to be immediate and/or modified release. Modified release formulations include delayed-, sustained-, pulsed-, controlled-, targeted and programmed release.

The compounds of the invention may also be administered directly to the eye or ear, typically in the form of drops of a micronised suspension or solution in isotonic, pH-adjusted, sterile saline. Other formulations suitable for ocular and aural administration include ointments, biodegradable (e.g. absorbable gel sponges, collagen) and non-biodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for
example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

The compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, *i*.*e*. as a carrier, diluent, or solubiliser. Most commonly used for these purposes are alpha-, beta- and gamma-cyclodextrins, examples of which may be found in International Patent Applications Nos. WO 91/11172, WO 94/02518 and WO 98/55148.

Inasmuch as it may desirable to administer a combination of active compounds, for example, for the purpose of treating a particular disease or condition, it is within the scope of the present invention that two or more pharmaceutical compositions, at least one of which contains a compound in accordance with the invention, may conveniently be combined in the form of a kit suitable for coadministration of the compositions.

Thus the kit of the invention comprises two or more separate pharmaceutical compositions, at least one of which contains a compound of formula (I) in accordance with the invention, and means for separately retaining said compositions, such as a container, divided bottle, or divided foil packet. An example of such a kit is the familiar blister pack used for the packaging of tablets, capsules and the like.

The kit of the invention is particularly suitable for administering different dosage forms, for example, oral and parenteral, for administering the separate compositions at different dosage intervals, or for titrating the separate compositions against one another. To assist compliance, the kit typically comprises directions for administration and may be provided with a so-called memory aid.

For administration to human patients, the total daily dose of the compounds of the invention is typically in the 0.001 mg to 2000 mg depending, of course, on the mode of administration. For example, oral administration may require a total daily dose of from 0.1 mg to 2000 mg, while an intravenous dose may only require from 0.01 mg to 100 mg. The total daily dose may be administered in single or divided doses and may, at the physician's discretion, fall outside of the typical range given herein.

These dosages are based on an average human subject having a weight of about 60 kg to 70 kg. The physician will readily be able to determine doses for subjects whose weight falls outside this range, such as infants and the elderly.

For the avoidance of doubt, references herein to "treatment" include references to curative, palliative and prophylactic treatment.

According to another embodiment of the present invention, the compounds of the formula (I) or pharmaceutically acceptable salts, derived forms or compositions thereof, can also be used as a combination with one or more additional therapeutic agents to be co-administered to a patient to obtain some particularly desired therapeutic end result. The second and more additional therapeutic agents may also be a compound of the formula (I) or a pharmaceutically acceptable salt, derived forms or compositions thereof, or one or more histamine H₄ receptor ligands known in the art. More typically, the second and more therapeutic agents will be selected from a different class of therapeutic agents.

As used herein, the terms "co-administration", "co-administered" and "in combination with", referring to the compounds of formula (I) and one or more other therapeutic agents, is intended to mean, and does refer to and include the following:
- simultaneous administration of such combination of compound(s) of formula (I) and therapeutic agent(s) to a patient in need of treatment, when such components are formulated together into a single dosage form which releases said components at substantially the same time to said patient,
- substantially simultaneous administration of such combination of compound(s) of formula (I) and therapeutic agent(s) to a patient in need of treatment, when such components are formulated apart from each other into separate dosage forms which are taken at substantially the same time by said patient, whereupon said components are released at substantially the same time to said patient,
- sequential administration of such combination compound(s) of formula (I) and therapeutic agents(s) to a patient in need of treatment, when such components are formulated apart from each other into separate dosage forms which are taken at consecutive times by said patient with a significant time interval between each administration, whereupon said components are released at substantially different times to said patient; and
- sequential administration of such combination of compound(s) of formula (I) and therapeutic agent(s) to a patient in need of treatment, when such components are formulated together into a single dosage form which releases said components in a controlled manner whereupon they are concurrently, consecutively, and/or overlapingly administered at the same and/or different times by said patient,
where each part may be administered by either the same or different route.

Suitable examples of other therapeutic agents which may be used in combination with the compound(s) of formula (I), or pharmaceutically acceptable salts, derived forms or compositions thereof, include, but are by no means limited to :
- Histamine H₁ receptor antagonists, for instance loratidine, desloratidine, fexofenadine and cetirizine
- Histamine H₃ receptor antagonists
- Histamine H₂ receptor antagonists
- Leukotriene antagonists, including antagonists of LTB₄, LTC₄, LTD₄, and LTE₄, in particular Montelukast
- Phosphodiesterase PDE4 inhibitors
- neurotransmitter re-uptake inhibitors, for instance fluoxetine, setraline, paroxetine, ziprasidone
- 5-Lipoxygenase (5-LO) inhibitors or 5-lipoxygenase activating protein (FLAP) antagonists,
- α₁- and α₂-adrenoceptor agonist vasoconstrictor sympathomimetic agents for decongestant use,
- Muscarinic M3 receptor antagonists or anticholinergic agents,
- β₂-adrenoceptor agonists,
- Theophylline,
- Sodium cromoglycate,
- COX-1 inhibitors (NSAIDs) and COX-2 selective inhibitors,
- Oral or inhaled Glucocorticosteroids,
- Monoclonal antibodies active against endogenous inflammatory entities,
- Anti-tumor necrosis factor (anti-TNF-α) agents,
- Adhesion molecule inhibitors including VLA-4 antagonists,
- Kinin-B₁ - and B₂ -receptor antagonists,
- Immunosuppressive agents,
- Inhibitors of matrix metalloproteases (MMPs),
- Tachykinin NK₁, NK₂ and NK₃ receptor antagonists,
- Elastase inhibitors,
- Adenosine A2a receptor agonists,
- Inhibitors of urokinase,
- Compounds that act on dopamine receptors, e.g. D2 agonists,
- Modulators of the NFκβ pathway, e.g. IKK inhibitors,
- Agents that can be classed as mucolytics or anti-tussive,
- antibiotics,
- p38 MAP kinase inhibitors
- Phosphodiesterase PDE5 inhibitors
- Prostaglandin D2 receptor antagonists (DP1 and CRTH2)
- Inhibition of Prostaglandin D synthase (PGDS)
- Inhibitors of phosphoinositide-3-kinase, and
- Inhibitors of Syk tyrosine kinase.

According to the present invention, combination of the compounds of formula (I) with:
- Histamine H₁ receptor antagonists, for instance loratidine, desloratidine, fexofenadine and cetirizine,
- Histamine H₃ receptor antagonists,
- Histamine H₂ receptor antagonists,
- Leukotriene antagonists, including antagonists of LTB₄, LTC₄, LTD₄, and LTE₄, in particular Montelukast and
- Phosphodiesterase PDE4 inhibitors are preferred.

The compounds of formula (I) have the ability to interact with the H₄ receptor and thereby have a wide range of therapeutic applications, as described further below, because of the essential role, which the H₄ receptor plays in the physiology of all mammals. According to this invention H₄ ligands are meant to include H₄ receptor antagonists, agonists and inverse agonists. For the preferred indications to be treated according to the invention, H₄ antagonists are believed to be most suitable.

Therefore, a further aspect of the present invention relates to the compounds of formula (I) or pharmaceutically acceptable salts, derived forms or compositions thereof, for use as medicaments, more particularly in the treatment of diseases, disorders, and conditions in which the H₄ receptor is involved. More specifically, the present invention also concerns the compounds of formula (I), or pharmaceutically acceptable salts, derived forms or compositions thereof, for use in the treatment of diseases, disorders, and conditions selected from the group consisting of:
- inflammatory diseases;
- respiratory diseases (e.g. adult respiratory distress syndrome, acute respiratory distress syndrome, bronchitis, chronic bronchitis, chronic obstructive pulmonary disease, cystic fibrosis, asthma, emphysema, rhinitis, chronic sinusitis), allergy, allergy-induced airway responses, allergic rhinitis, viral rhinitis, non-allergic rhinitis, perennial and seasonal rhinitis, nasal congestion, allergic congestion;
- female and male sexual dysfunction;
- skin diseases such as dermatitis and psoriasis;
- cardiac dysfunctions such as myocardial ischaemia and arrythmia;
- diseases of the gastrointestinal tract such as inflammatory bowel disease, Crohn's disease and colitis ulcerosa;
- cancer;
- rheumatoid arthritis;
- hypotension;
- pain and
- overactive bladder conditions.

The compounds of formula (I) according to the present invention are particularly suitable for the treatment of asthma, allergy, allergy-induced airway responses, allergic rhinitis, viral rhinitis, non-allergic rhinitis, perennial and seasonal rhinitis, nasal congestion, allergic congestion.

A still further aspect of the present invention also relates to the use of the compounds of formula (I), or pharmaceutically acceptable salts, derived forms or compositions thereof, for the manufacture of a drug being a H₄ ligand. In particular, the present inventions concerns the use of the compounds of formula (I), or pharmaceutically acceptable salts, derived forms or compositions thereof, for the manufacture of a drug for the treatment of H₄ mediated diseases and/or conditions, in particular the diseases and/or conditions listed above.

As a consequence, the present invention provides a particularly interesting method to treat a mammal, including a human being, with an effective amount of a compound of formula (I), or a pharmaceutically acceptable salt, derived form or composition thereof. More precisely, the present invention provides a particularly interesting method for the treatment of a H₄ mediated diseases and/or conditions in a mammal, including a human being, in particular the diseases and/or conditions listed above, comprising administering to said mammal an effective amount of a compound of formula (I), its pharmaceutically acceptable salts and/or derived forms.

The following examples illustrate the preparation of compounds of formula (I) according to the present invention

¹H Nuclear magnetic resonance (NMR) spectra were in all cases consistent with the proposed structures. Characteristic chemical shifts (δ) are given in parts-per-million (ppm) downfield from tetramethylsilane using conventional abbreviations for designation of major peaks: e.g. s (singlet), d (doublet), t (triplet), q (quartet) m (multiplet) and br (broad). The mass spectra (m/z) were recorded using either electrospray ionisation (ESI) or atmospheric pressure chemical ionisation (APCI).

### EXAMPLES SECTION

### Preparation 1: (5-Mothoxy-1H-benzimidazol-2-yl)methanol

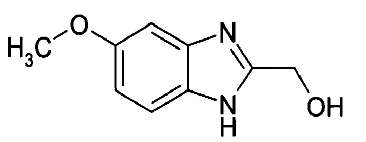

A solution of 4-methoxybenzene-1,2-diamine (1.13g, 8.18mmol) and hydroxyacetic acid (1.85g, 24.5mmol) in 6N hydrochloric acid (25ml) was heated at reflux for 16 hours. The reaction mixture was cooled to room temperature and neutralised by addition of solid sodium hydroxide. The resulting precipitate was filtered off and dried *in vacuo* to give the title compound as a pale yellow solid (638mg).
¹H NMR (400MHz, CD₃OD): δ 7.40 (1 H, d), 7.04 (1 H, d), 6.84 (1 H, dd), 4.78 (2H, s), 3.82 (3H, s) ppm.
MS (APCI) m/z 179 [M+H]⁺, 177 [M-H]⁻

### Preparation 2: (5-Fluoro-1H-benzimidazol-2-yl)methanol

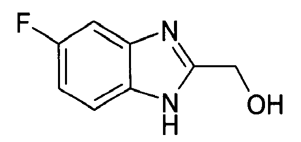

Prepared from 4-fluorobenzene-1,2-diamine using the method of preparation 1 to give the title compound as a brown crystalline solid.
¹H NMR (400MHz, CD₃OD): δ 7.48 (1H, dd), 7.22 (1H, dd), 7.01-6.96 (1H, m), 4.80 (2H, s) ppm.
MS (APCI) m/z 167 [M+H]⁺, 165 [M-H]⁻

### Preparation 3: (5-Methyl-1H-benzimidazol-2-yl)methanol

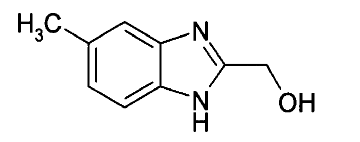

Prepared from 4-methyl-1,2-diamine using the method of preparation 1 to give the title compound as a pale yellow solid.
¹H NMR (400MHz, CD₃OD): δ 7.40 (1 H, d), 7.32 (1 H, s), 7.04 (1 H, dd), 4.79 (2H, s), 2.43 (3H, s) ppm.
MS (APCI) m/z 163 [M+H]⁺, 161 [M-H]⁻

### Preparation 4: (5-Chloro-1H-benzimidazol-2-yl)methanol

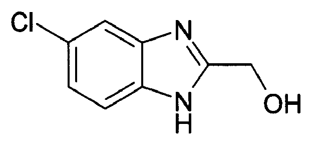

Prepared from 4-chloro-1,2-diamine using the method of preparation 1 to give the title compound as a pale yellow solid.
¹H NMR (400MHz, DMSO_{d6}): δ 12.53-12.48 (1H, m), 7.60-7.45 (3H, m), 7.19-7.17 (1 H, m), 5.76 (1 H, t), 4.71 (2H, d) ppm.
MS (APCI) m/z 183/185 [M+H]⁺, 181/183 [M-H]⁻

### Preparation 5: 5-Methoxy-1H-benzimidazole-2-carboxylic acid

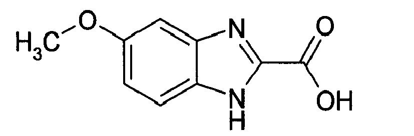

A suspension of (5-Methoxy-1*H*-benzimidazol-2-yl)methanol (638mg, 3.58mmol) in 1 N aqueous sodium hydroxide (0.95ml) and water (40ml) was heated to reflux and a solution of potassium permanganate (848mg, 5.36mmol) in water was added drop wise over a period of 30 minutes. The reaction mixture was refluxed for a further hour and then cooled to room temperature. The reaction mixture was acidified to pH4 by addition of acetic acid and the resulting solid filtered and dried in vacuo to give the title compound as a pale yellow solid (332mg).
¹H NMR (400MHz, CD₃OD): δ 7.58 (1 H, d), 7.05 (s, 1H), 7.00 (1 H, dd), 3.83 (3H, s) ppm.

### Preparation 6: 5-Fluoro-1H-benzimidazole-2-carboxylic acid

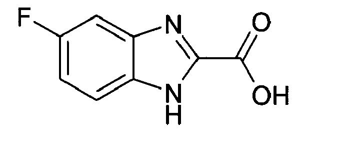

Prepared from the alcohol of preparation 2 using the method of preparation 5 to give the title compound as a pale brown solid.
¹H NMR (400MHz, DMSO_{d6}): δ 12.54 (1 H, bs), 8.26 (1 H, bs), 7.57 (1 H, bs), 7.40 (1 H, bs), 7.06 (1 H, bs) ppm.

### Preparation 7: 5-Methyl-1H-benzimidazole-2-carboxylic acid

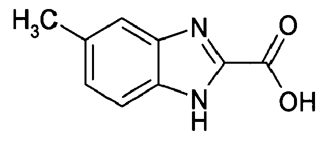

Prepared from the alcohol of preparation 3 using the method of preparation 5 to give the title compound as a pale yellow solid.
¹H NMR (400MHz, DMSO_{d6}): δ 7.56 (1 H, d), 7.44 (1 H, s), 7.23 (1 H, d), 2.46 (3H, s) ppm.

### Preparation 8: 5-Chloro-1H-benzimidazole-2-carboxylic acid

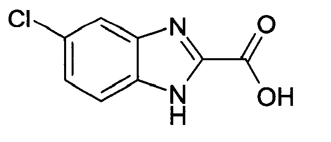

Prepared from the alcohol of preparation 4 using the method of preparation 5 to give the title compound as a pale yellow solid.
¹H NMR (400MHz, DMSO_{d6}): δ 7.71-7.67 (2H, m), 7.37 (1 H, d) ppm.

### Preparation 9: 6,7-Dimethyl-2-(trichloromethyl)-1H-benzimidazole

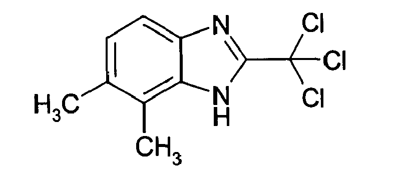

A solution of 3,4-dimethylbenzene-1,2-diamine (1.0g, 7.34mmol) in acetic acid (10ml) was treated with methyl-2,2,2-trichloroacetimidate (1.0ml, 8.07mmol) and the resulting solution was left to stir at room temperature for 16 hours. The reaction mixture was poured onto ice (∼100g) and then extracted with dichloromethane (100ml). The organic phase was separated, dried (sodium sulfate) and the solvent removed *in vacuo*. The residue was triturated with diethyl ether and the title compound isolated by filtration (360mg) as a brown solid. The material was used crude in the next step with no further characterization.

### Preparation 10: 6,7-Difluoro-2-(trichloromethyl)-1H-benzimidazole

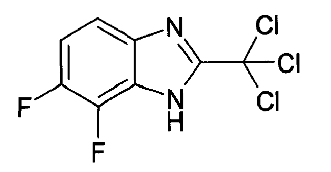

A solution of 3,4-difluorobenzene-1,2-diamine (274mg, 1.90mmol) in acetic acid (4ml) was treated with methyl-2,2,2-trichloroacetimidate (0.25ml, 2.09mmol) and the resulting solution was left to stir at room temperature for 16 hours. The reaction mixture was poured onto ice (~50g) and the resulting precipitate was filtered and dried *in vacuo* to give the title compound as a borwn solid (496mg). The material was used crude in the next step with no further characterization.

### Preparation 11: 7-Methyl-2-(trichloromethyl)-1H-benzimidazole

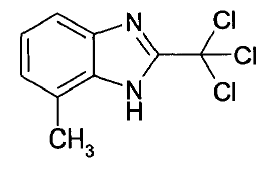

Prepared from 3-methylbenzene-1,2-diamine using the method of preparation 10 to give the title compound as a brown solid. The material was used crude in the next step with no further characterization.

### Preparation 12: 5,6-Difluoro-2-(trichloromethyl)-1H-benzimidazole

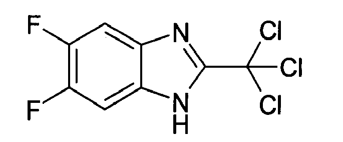

Prepared from 5,6-difluorobenzene-1,2-diamine using the method of preparation 10 to give the title compound as a brown solid. The material was used crude in the next step with no further characterization.

### Preparation 13: 5,7-Difluoro-2-(trichloromethyl)-1H-benzimidazole

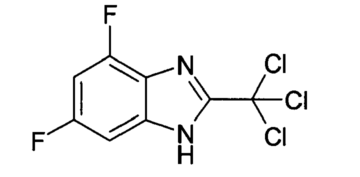

Prepared from 3,5-difluorobenzene-1,2-diamine using the method of preparation 10 to give the title compound as a brown solid. The material was used crude in the next step with no further characterization.

### Preparation 14: 5-Chloro-7-methyl-2-(trichloromethyl)-1H-benzimidazole

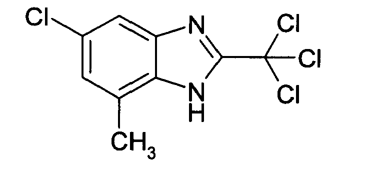

Prepared from 5-chloro-3-methylbenzene-1,2-diamine using the method of preparation 10 to give the title compound as a yellow solid. The material was used crude in the next step with no further characterization.

### Preparation 15: 7-Chloro-5-methyl-2-(trichloromethyl)-1H-benzimidazole

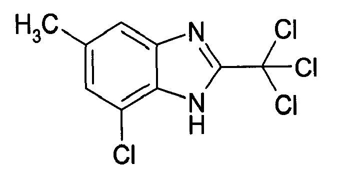

Prepared from 3-chloro-5-methylbenzene-1,2-diamine using the method of preparation 10 to give the title compound as a brown solid. The material was used crude in the next step with no further characterization.

### Preparation 16: 5-Iodo-2-(trichloromethyl)-1H-benzimidazole

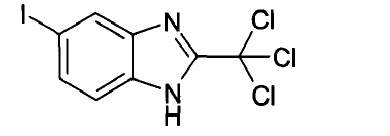

Prepared from 4-iodobenzene-1,2-diamine using the method of preparation 10 to give the title compound as a brown solid. The material was used crude in the next step.
¹H NMR (400MHz, CDCl₃): δ 8.03 (1 H, s), 7.64 (1 H, d), 7.45 (1 H, d) ppm.

### Preparation 17: Methyl 2-(trichloromethyl)-1H-benzimidazole-5-carboxylate

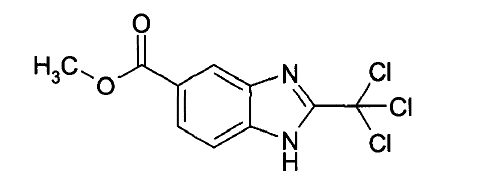

Prepared from methyl 3,4-diaminobenzoate using the method of preparation 10 to give the title compound as a brown solid. The material was used crude in the next step.
¹H NMR (400MHz, CDCl₃): δ 8.45 (1 H, s), 8.07 (1 H, d), 7.70 (1 H, d), 3.95 (3H, s) ppm.

### Preparation 18: 5-Chloro-6-fluoro-2-(trichloromethyl)-1H-benzimidazole

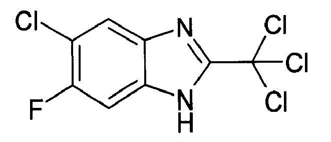

Prepared from 4-chloro-5-fluorobenzene-1,2-diamine using the method of preparation 10 to give the title compound as a brown solid. The material was used crude in the next step with no further characterisation.

### Preparation 19: 5,6-Dimethyl-2-(trichloromethyl)-1H-benzimidazole

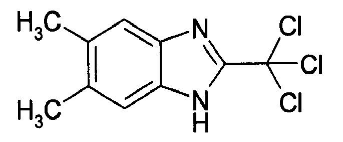

Prepared from 4,5-dimethylbenzene-1,2-diamine using the method of preparation 10 to give the title compound as a brown solid. The material was used crude in the next step with no further characterisation.

### Preparation 20: 5,6-Dichloro-2-(trichloromethyl)-1H-benzimidazole

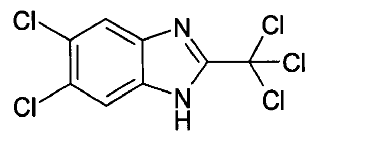

Prepared from 4,5-dichlorobenzene-1,2-diamine using the method of preparation 10 to give the title compound as a brown solid. The material was used crude in the next step with no further characterisation.

### Preparation 21: 2-(Trichloromethyl)-5-(trifluoromethyl)-1H-benzimidazole

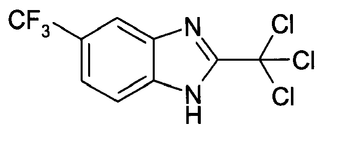

Prepared from 4-(trifluoromethyl)benzene-1,2-diamine using the method of preparation 10 to give the title compound as a pale yellow solid. The material was used crude in the next step.
¹H NMR (400MHz, CDCl₃): δ 8.05 (1 H, s), 7.87 (1 H, d), 7.71 (1 H, d) ppm.

### Preparation 22: 4-Chloro-6-fluoro-2-(trichloromethyl)-1H-benzimidazole

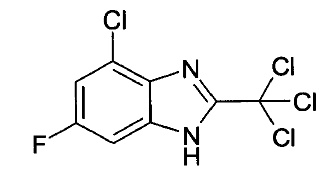

Prepared from 3-chloro-5-fluorobenzene-1,2-diamine using the method of preparation 10 to give the title compound as an orange solid. The material was used crude in the next step with no further characterization.

### Preparation 23: 4,6-Dichloro-2-(trichloromethyl)-1H-benzimidazole

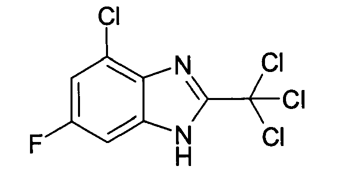

Prepared from 3,5-dichlorobenzene-1,2-diamine using the method of preparation 10 to give the title compound as a yellow solid. The material was used crude in the next step with no further characterization.

### Preparation 24: 5-Phenyl-2-(trichloromethyl)-1H-benzimidazole

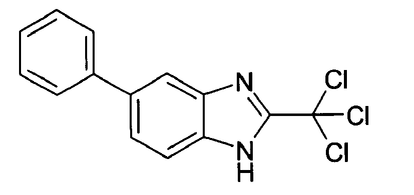

Prepared from biphenyl-3,4-diamine using the method of preparation 10 to give the title compound as a brown oil. The material was used crude in the next step with no further characterization.

### Preparation 25: 5-Fluoro-4-methyl-2-(trichloromethyl)-1H-benzimidazole

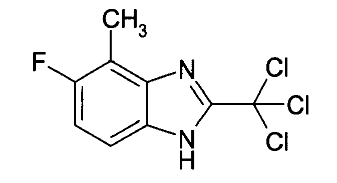

Prepared from 4-fluoro-3-methylbenzene-1,2-diamine using the method of preparation 10 to give the title compound as a brown oil. The material was used crude in the next step with no further characterization.

### Example 1: 2-[(5-Methylhexahydropyrrolo[3,4-c]pyrrol-2(1H)-yl)carbonyl]-1H-benzimidazole

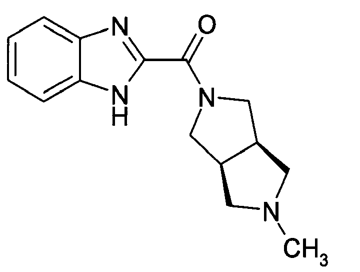

A solution of 1*H*-benzimidazole-2-carboxylic acid (456mg, 2.81 mmol) in N,N-dimethylacetamide (5ml) was treated with (3a*R*,6a*S*)-2-methyloctahydropyrrolo[3,4-c]pyrrole (322mg, 2.55mmol), triethylamine (0.71 ml, 5.11mmol) and O-benzotriazo-1-yl-*N,N,N'N'*-tetramethyluronium hexafluorophosphate (1.16g, 3.06mmol) and the resulting solution left to stir at room temperature for 16 hours. The reaction mixture was purified directly by SCX resin, eluting non-basic compounds with methanol and the basic compounds with 1 N ammonia in methanol. The basic washings were concentrated *in vacuo* and purified by flash column chromatography on silica gel eluting with dichloromethane : methanol : 880 ammonia (99:1:0.1 changing to 90:10:1, by volume) to give the title compound as a pale yellow solid (499mg).
¹H NMR (400MHz, CD₃OD): δ 7.71-7.69 (2H, m), 7.39-7.34 (2H, m), 4.42-4.30 (2H, m), 3.95-3.77 (2H, m), 3.15-2.98 (2H, m), 2.84-2.77 (2H, m), 2.56-2.52 (2H, m), 2.37 (3H, s) ppm.

MS (electrospray): m/z 271 [M+H]⁺, 269 [M-H]⁻

### Example 2: 5-Methoxy-2-{[(3aR,6aS)-5-methylhexahydropyrrolo[3,4-c]pyrrol-2(1H)-yl]carbonyl}-1H-benzimidazole

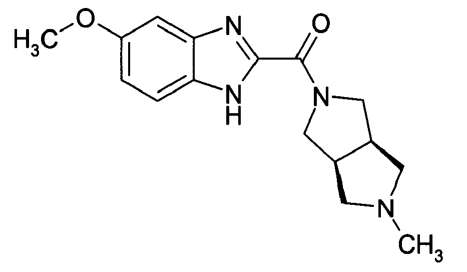

Prepared from 5-methoxy-1*H*-benzimidazole-2-carboxylic acid (preparation 5) using the method from example 1 to give the title compound as a pale brown solid.
¹H NMR (400MHz, CD₃OD): δ 7.55 (1 H, d), 7.09 (1 H, s), 6.95 (1 H, d), 4.36-4.24 (2H, m), 3.89-3.71 (5H, m), 3.10-2.89 (2H, m), 2.79-2.72 (2H, m), 2.49-2.46 (2H, m), 2.32 (3H, s) ppm.
MS (APCI) m/z 301 [M+H]⁺, 299 [M-H]⁻

### Example 3: 5-Fluoro-2-{[(3aR,6aS)-5-methylhexahydropyrrolo[3,4-c]pyrrol-2(1H)-yl]carbonyl}-1H-benzimidazole

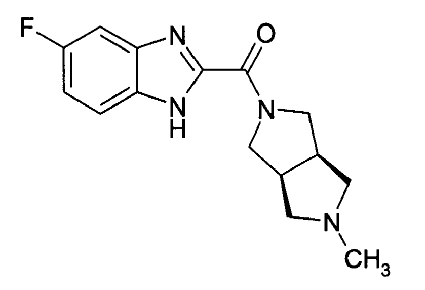

Prepared from 5-fluoro-1*H*-benzimidazole-2-carboxylic acid (preparation 6) using the method from example 1 to give the title compound as a pale brown solid.
¹H NMR (400MHz, CD₃OD): δ 7.65 (1H, dd), 7.33 (1H, dd), 7.13-7.07 (1H, m), 4.37-4.26 (2H, m), 3.90-3.74 (2H, m), 3.10-2.92 (2H, m), 2.79-2.71 (2H, m), 2.51-2.48 (2H, m), 2.32 (3H, s) ppm.
MS (APCI) m/z 289 [M+H]⁺, 287 [M-H]⁻

### Example 4: 5-Methyl-2-{[(3aR,6aS)-5-methylhexahydropyrrolo[3,4-c]pyrrol-2(1H)-yl]carbonyl}-1H-benzimidazole

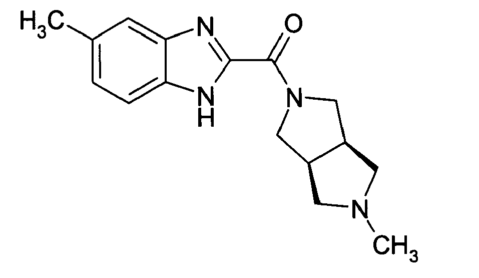

Prepared from 5-methyl-1*H*-benzimidazole-2-carboxylic acid (preparation 7) using the method from example 1 to give the title compound as a pale yellow solid.
¹H NMR (400MHz, CD₃OD): δ 7.54 (1 H, d), 7.42 (1 H, s), 7.15 (1 H, d), 4.35-4.23 (2H, m), 3.89-3.72 (2H, m), 3.11-2.91 (2H, m), 2.79-2.71 (2H, m), 2.50-2.47 (2H, m), 2.32 (3H, s) ppm.
MS (APCI) m/z 285 [M+H]⁺, 283 [M-H]⁻

### Example 5: 5-Chloro-2-{[(3aR,6aS)-5-methylhexahydropyrrolo[3,4-c]pyrrol-2(1H)-yl]carbonyl}-1H-benzimidazole

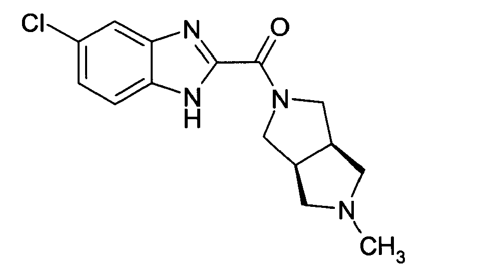

Prepared from 5-chloro-1*H*-benzimidazole-2-carboxylic acid (preparation 8) using the method from example 1 to give the title compound as a pale yellow solid.
¹H NMR (400MHz, CD₃OD): δ 7.64 (1 H, d), 7.60 (1 H, s), 7.29 (1 H, d), 4.37-4.25 (2H, m), 3.88-3.71 (2H, m), 3.11-2.91 (2H, m), 2.78-2.70 (2H, m), 2.51-2.48 (2H, m), 2.32 (s, 3H) ppm.
MS (APCI) m/z 305/307 [M+H]⁺, 303/305 [M-H]⁻

### Example 6: 5-chloro-2-[(3aR,6aS)-hexahydropyrrolo[3,4-c]pyrrol-2(1H)-ylcarbonyl]-1H-benzimidazole

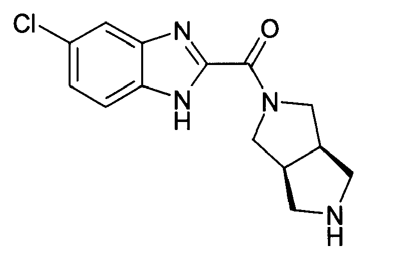

A solution of 5-chloro-1*H*-benzimidazole-2-carboxylic acid (preparation 8) (100mg, 0.50mmol) in N,N-dimethylacetamide (5ml) was treated with *tert-*butyl (3a*R*,6a*S*)-hexahydropyrrolo[3,4-*c*]pyrrole-2(1*H*)-carboxylate (108mg, 0.51 mmol) and O-benzotriazo-1-yl-*N,N,N'N*'-tetramethyluronium hexafluorophosphate (256mg, 0.67mmol) and the resulting solution left to stir at room temperature for 16 hours. The reaction mixture was diluted with ethyl acetate (20ml) and water (20ml) and the organic phase separated, dried (sodium sulphate) and the solvent reduced in vacuo. The residue was treated with a 1 M solution of HCl in methanol (15ml) and left to stir at room temperature for 16 hours. The solvent was removed in vacuo and the residue purified by flash column chromatography on silica gel eluting with dichloromethane : methanol : 880 ammonia (99:1:0.1 changing to 90:10:1, by volume) to give the title compound as a colourless solid (50mg).
¹H NMR (400MHz, CD₃OD): δ 7.69-7.65 (2H, m), 7.34(1H, d), 4.45-4.40 (1 H, m), 4.24-4.20 (1 H, m), 3.99-3.94 (1 H, m), 3.71-3.66 (1 H, m), 3.19-3.11 (2H, m), 3.09-2.92 (2H, m), 2.83-2.79 (2H, m) ppm.
MS (APCI) m/z 291/293 [M+H]⁺, 289/291 [M-H]⁻

### Example 7: 5-Chloro-2-{[(3aR,6aS)-5-methylhexahydropyrrolo[3,4-c]pyrrol-2(1H)-yl]carbonyl}-1H-indole

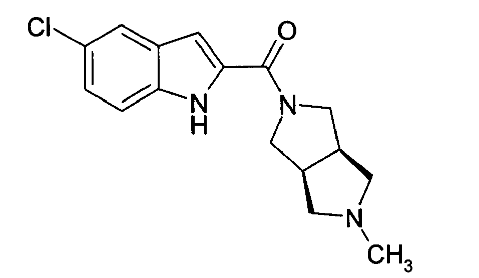

Prepared from 5-chloro-1*H*-indole-2-carboxylic acid according to the method of example 1 to give the title compound as a colourless solid.
¹H NMR (400MHz, CD₃OD): δ 7.67 (1 H, s), 7.46 (1 H, d), 7.24 (1 H, dd), 6.97 (1H, s), 4.20-3.73 (4H, m), 3.17-2.96 (2H, m), 2.82-2.75 (2H, m), 2.61-2.54 (2H, m), 2.38 (3H, s) ppm.
MS (APCI) m/z 304/306 [M+H]⁺, 302/304 [M-H]⁻

### Example 8: 5-Chloro-2-{[(3aR,6aS)-hexahydropyrrolo[3,4-c]pyrrol-2(1H)-yl]carbonyl}-1H-indole

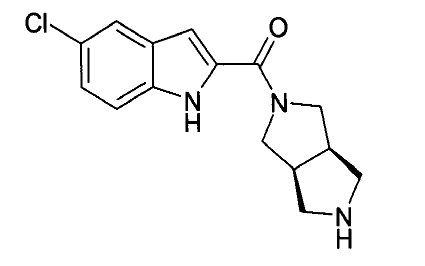

A solution of 5-chloro-1*H*-indole-2-carboxylic acid (100mg, 0.51mmol) in N,N-dimethylacetamide (5ml) was treated with *tert*-butyl (3a*R*,6a*S*)-hexahydropyrrolo[3,4-*c*]pyrrole-2(1 *H*)-carboxylate (108mg, 0.51mmol) and O-benzotriazo-1-yl-*N,N,N'N*'-tetramethyluronium hexafluorophosphate (256mg, 0.67mmol) and the resulting solution left to stir at room temperature for 16 hours. The reaction mixture was diluted with ethyl acetate (20ml) and water (20ml) and the organic phase separated, dried (sodium sulphate) and the solvent reduced in vacuo. The residue was treated with a 1 M solution of HCl in methanol (15ml) and left to stir at room temperature for 16 hours. The solvent was removed in vacuo and the residue purified by flash column chromatography on silica gel eluting with dichloromethane : methanol : 880 ammonia (99:1:0.1 changing to 90:10:1, by volume) to give the title compound as a pale yellow solid (42mg).
¹H NMR (400MHz, DMSO_{d6}): δ 11.77 (1 H, bs), 7.69 (1 H, bs), 7.45 (1H, d), 7.20 (1 H, d), 6.93 (1 H, s), 4.13-3.45 (4H, m), 2.99-2.63 (6H, m), 2.52 (3H, s) ppm (poorly resolved spectrum).
MS (APCI) m/z 290/292 [M+H]⁺, 288/290 [M-H]⁻

### Example 9: 5-Methoxy-3-methyl-2-{[(3aR,6aS)-5-methylhexahydropyrrolo[3,4-c]pyrrol-2(1H)-yl]carbonyl}-1H-indole

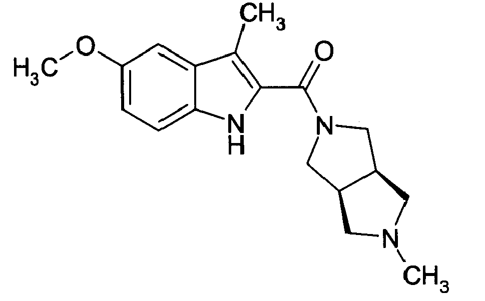

Prepared from 5-methoxy-3-methyl-1*H*-indole-2-carboxylic acid according to the method of example 1 to give the title compound as a pale yellow solid.
¹H NMR (400MHz, CDCl₃): δ 9.42 (1 H, bs), 7.28 (1 H, d), 6.99 (1 H, s), 6.92 (1 H, d), 3.88-3.58 (7H, m), 2.91-2.78 (2H, m), 2.56-2.46 (4H, m), 2.37 (3H, s), 2.32 (3H, s) ppm.
MS (APCI) m/z 314 [M+H]⁺, 312 [M-H]⁻

### Example 10: 5-(Benzyloxy)-2-{[(3aR,6aS)-5-methylhexahydropyrrolo[3,4-c]pyrrol-2(1H)-yl]carbonyl}-1H-indole

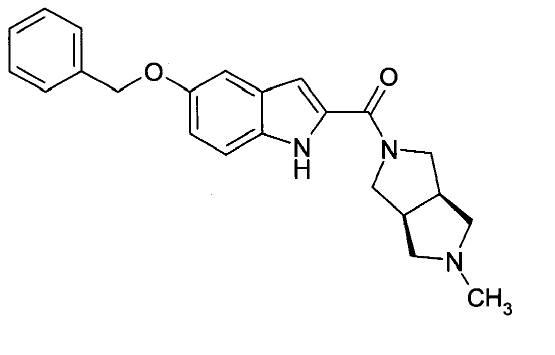

Prepared from 5-(benzyloxy)-3-methyl-1 H-indole-2-carboxylic acid according to the method of example 1 to give the title compound as a colourless solid.
¹H NMR (400MHz, CDCl₃): δ 9.39 (1 H, bs), 7.47 (2H, d), 7.39 (2H, dd), 7.30 (2H, d), 7.13 (1 H, s), 7.05 (1 H, d), 6.76 (1 H, s), 5.10 (2H, s), 4.15-4.03 (2H, m), 3.83-3.72 (2H, m), 3.11-2.91 (2H, m), 2.75-2.69 (2H, m), 2.59-2.54 (2H, m), 2.38 (s, 3H) ppm.
MS (APCI) m/z 376 [M+H]⁺

### Example 11: 6-Methyl-2-{[(3aR,6aS)-5-methylhexahydropyrrolo[3,4-c]pyrrol-2(1H)-yl]carbonyl}-1H-indole

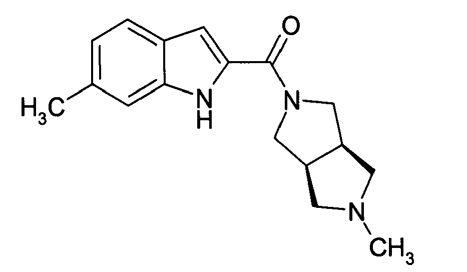

Prepared from 5-methyl-1*H*-indole-2-carboxylic acid according to the method of example 1 to give the title compound as a pale yellow solid.
¹H NMR (400MHz, CDCl₃): δ 9.69 (1 H, bs), 7.54 (1 H, d), 7.23 (1 H, s), 6.98 (1H, d), 6.81 (1H, s), 4.19-4.02 (2H, m), 3.86-3.72 (2H, m), 3.11-2.85 (2H, m), 2.68-2.64 (2H, m), 2.57-2.55 (2H, m), 2.47 (3H, s), 2.35 (3H, s) ppm.
MS (APCI) m/z 284 [M+H]⁺, 282 [M-H]⁻

### Example 12: 4-Methoxy-2-{[(3aR,6aS)-5-methylhexahydropyrrolo[3,4-c]pyrrol-2(1H)-yl]carbonyl}-1H-indole

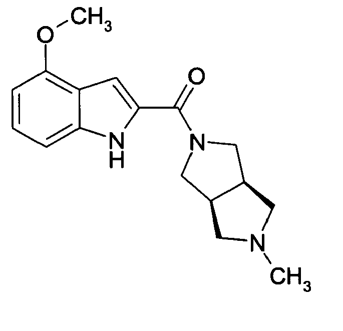

Prepared from 4-methoxy-1*H*-indole-2-carboxylic acid according to the method of example 1 to give the title compound as a colourless solid.
¹H NMR (400MHz, CDCl₃): δ 9.38 (1 H, bs), 7.21 (1 H, t), 7.05 (1 H, d), 6.96 (1 H, s), 6.51 (1 H, s), 4.14-4.04 (2H, m), 3.97 (3H, s), 3.89-3.78 (2H, m), 3.12-3.00 (2H, m), 2.90-2.75 (2H, m), 2.60-2.57 (2H, m), 2.42 (3H, s) ppm.
MS (APCI) m/z 300 [M+H]⁺, 298 [M-H]⁻

### Example 13: 6-Chloro-2-{[(3aR,6aS)-5-methylhexahydropyrrolo[3,4-c]pyrrol-2(1H)-yl]carbonyl}-1H-indole

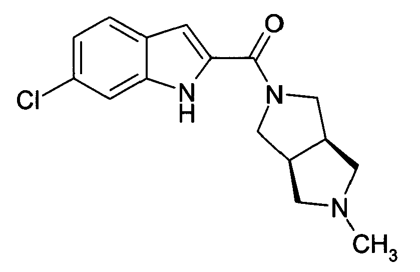

Prepared from 6-chloro-1*H*-indole-2-carboxylic acid according to the method of example 1 to give the title compound as a pale yellow solid.
¹H NMR (400MHz, CDCl₃): δ 10.24 (1 H, bs), 7.55 (1 H, d), 7.46 (1 H, s), 7.06 (1 H, d), 6.81 (1H, s), 4.16-4.08 (m, 2H), 3.83-3.76 (m, 2H), 3.14-2.91 (m, 2H), 2.68-2.59 (4H, m), 2.36 (s, 3H) ppm.
MS (APCI) m/z 304/306 [M+H]⁺, 302/304 [M-H]⁻

### Example 14: 5-Methoxy-2-{[(3aR,6aS)-5-methylhexahydropyrrolo[3,4-c]pyrrol-2(1H)-yl]carbonyl}-1H-indole

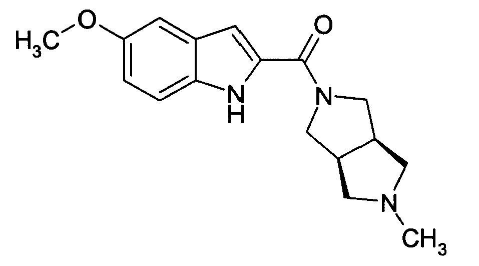

Prepared from 5-methoxy-1*H*-indole-2-carboxylic acid according to the method of example 1 to give the title compound as a colourless solid.
¹H NMR (400MHz, CDCl₃): δ 9.68 (1 H, bs), 7.34 (1 H, d), 7.06 (1 H, s), 6.96 (1H, dd), 6.77 (1H, s), 4.15-4.02 (2H, m), 3.85 (3H, s), 3.83-3.75 (2H, m), 3.11-2.91 (2H, m), 2.71-2.65 (2H, m), 2.59-2.53 (2H, m), 2.35 (3H, s) ppm.
MS (APCI) m/z 300 [M+H]⁺, 298 [M-H]⁻

### Example 15: 2-{[(3aR,6aS)-5-Methylhexahydropyrrolo[3,4-c]pyrrol-2(1H)-yl]carbonyl}-1H-indol-5-ol

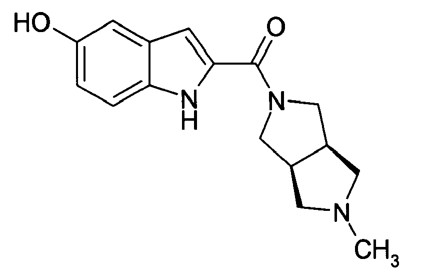

Prepared from 5-hydroxy-1*H*-indole-2-carboxylic acid according to the method of example 1 to give the title compound as a colourless solid.
¹H NMR (400MHz, CD₃OD): δ 7.33 (1 H, d), 7.02 (1 H, d), 6.86 (1 H, dd), 6.83 (1H, s), 4.20-3.72 (4H, m), 3.17-2.95 (2H, m), 2.83-2.79 (2H, m), 2.57-2.54 (2H, m), 2.39 (s, 3H) ppm.
MS (APCI) m/z 286 [M+H]⁺, 284 [M-H]⁻

### Example 16: 5-Fluoro-2-{[(3aR,6aS)-5-methylhexahydropyrrolo[3,4-c]pyrrol-2(1H)-yl]carbonyl}-1H-indole

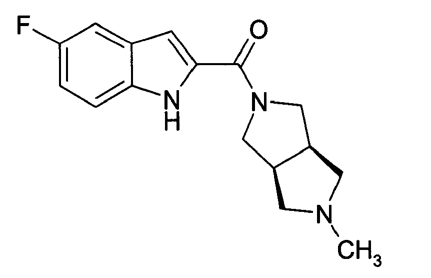

Prepared from 5-fluoro-1*H*-indole-2-carboxylic acid according to the method of example 1 to give the title compound as a colourless solid.
¹H NMR (400MHz, CDCl₃): δ 7.37 (1H, dd), 7.28 (1H, dd), 7.08-7.02 (1H, m), 6.81 (1H, s), 4.14-4.02 (2H, m), 3.84-3.74 (2H, m), 3.09-2.95 (2H, m), 2.74-2.70 (2H, m), 2.59-2.56 (2H, m), 2.37 (3H, s) ppm.
MS (APCI) m/z 288 [M+H]⁺, 286 [M-H]⁻

### Example 17: 5-Methyl-2-{[(3aR,6aS)-5-methylhexahydropyrrolo[3,4-c]pyrrol-2(1H)-yl]carbonyl}-1H-indole

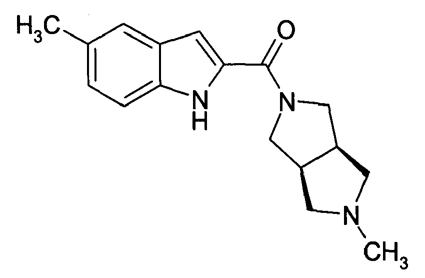

Prepared from 5-methyl-1*H*-indole-2-carboxylic acid according to the method of example 1 to give the title compound as a colourless solid.
¹H NMR (400MHz, CDCl₃): δ 7.43 (1H, s), 7.33 (1H, d), 7.12 (1 H, d), 6.77 (1H, s), 4.15-4.03 (2H, m), 3.84-3.74 (2H, m), 3.09-2.94 (2H, m), 2.80-2.71 (2H, m), 2.60-2.55 (2H, m), 2.44 (3H, s), 2.38 (3H, s) ppm.
MS (APCI) m/z 284 [M+H]⁺, 282 [M-H]⁻

### Example 18: 3,5-Dimethyl-2-{[(3aR,6aS)-5-methylhexahydropyrrolo[3,4-c]pyrrol-2(1H)-yl]carbonyl}-1H-indole

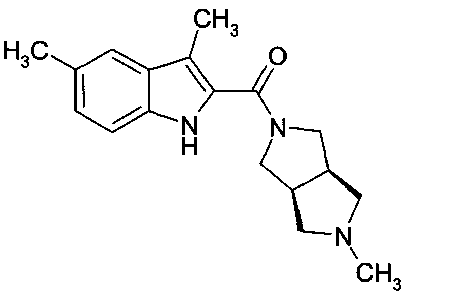

Prepared from 3,5-dimethyl-1*H*-indole-2-carboxylic acid according to the method of example 1 to give the title compound as a pale yellow solid.
¹H NMR (400MHz, CD₃OD): δ 7.32 (1 H, s), 7.23 (1 H, d), 7.03 (1 H, d), 3.79-3.74 (2H, m), 3.65-3.50 (2H, m), 2.95-2.90 (2H, m), 2.79-2.67 (2H, m), 2.41-2.33 (11 H, m) ppm.
MS (APCI) m/z 298 [M+H]⁺, 296 [M-H]⁻

### Example 19: 5-Chloro-3-methyl-2-{[(3aR,6aS)-5-methylhexahydropyrrolo[3,4-c]pyrrol-2(1H)-yl]carbonyl}-1H-indole

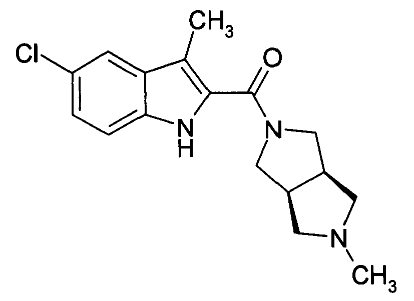

Prepared from 5-chloro-3-methyl-1*H*-indole-2-carboxylic acid according to the method of example 1 to give the title compound as a colourless solid.
¹H NMR (400MHz, CD₃OD): δ 7.53 (1H, s), 7.32 (1 H, d), 7.15 (1 H, d), 3.82-3.46 (4H, m), 2.98-2.89 (m, 2H), 2.76-2.66 (2H, m), 2.43-2.34 (2H, m), 2.33 (3H, s), 2.32 (3H, s) ppm.
MS (APCI) m/z 318/320 [M+H]⁺, 316/318 [M-H]⁻

### Example 20: 1-Methyl-2-{[(3aR,6aS)-5-methylhexahydropyrrolo[3,4-c]pyrrol-2(1H)-yl]carbonyl}-1H-indole

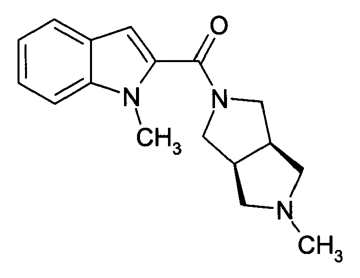

Prepared from 1-methyl-1*H*-indole-2-carboxylic acid according to the method of example 1 to give the title compound as a colourless solid.
¹H NMR (400MHz, CD₃OD): δ 7.60 (1 H, d), 7.42 (1 H, d), 7.28 (1 H, t), 7.10 (1 H, t), 6.76 (1 H, s), 3.89-3.83 (5H, m), 3.73-59 (2H, m), 2.96-2.89 (2H, m), 2.79-2.56 (2H, m), 2.55-2.33 (2H, m), 2.32 (3H, m) ppm.
MS (APCI) m/z 284 [M+H]⁺

### Example 21: 5-Bromo-2-{[(3aR,6aS)-5-methylhexahydropyrrolo(3,4-c]pyrrol-2(1H)-yl]carbonyl}-1H-indole

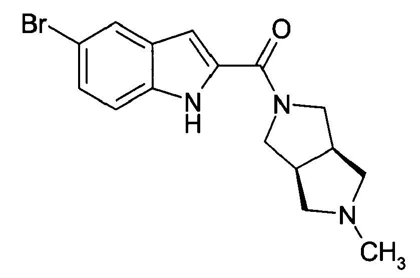

Prepared from 5-bromo-1*H*-indole-2-carboxylic acid according to the method of example 1 to give the title compound as a colourless solid.
¹H NMR (400MHz, CD₃OD): δ 7.79 (1 H, s), 7.37 (1 H, d), 7.31 (1 H, dd), 6.92 (1H, s), 4.18-3.72 (4H, m), 3.15-2.96 (2H, m), 2.86-2.78 (2H, m), 2.62-2.54 (2H, m), 2.39 (3H, s) ppm.
MS (APCI) m/z 348/350 [M+H]⁺, 346/348 [M-H]⁻

### Example 22: 7-Fluoro-2-{[(3aR,6aS)-5-methylhexahydropyrrolo[3,4-c]pyrrol-2(1H)-yl]carbonyl}-1H-indole

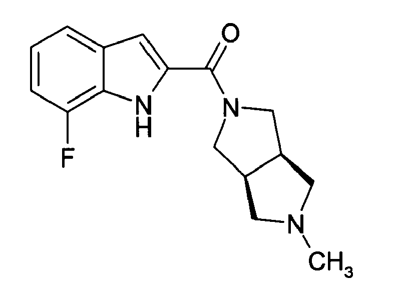

Prepared from 7-fluoro-1*H*-indole-2-carboxylic acid according to the method of example 1 to give the title compound as a colourless solid.
¹H NMR (400MHz, CD₃OD): δ 7.47 (1H, d), 7.08-6.97 (3H, m), 4.18-3.68 (4H, m), 3.16-2.92 (2H, m), 2.79-2.75 (2H, m), 2.57-2.53 (2H, m), 2.38 (3H, s) ppm.
MS (APCI) m/z 288 [M+H]⁺, 286 [M-H]⁻

### Example 23: 5,6-Dimethoxy-2-{[(3aR,6aS)-5-methylhexahydropyrrolo[3,4-c]pyrrol-2(1H)-yl]carbonyl}-1H-indole

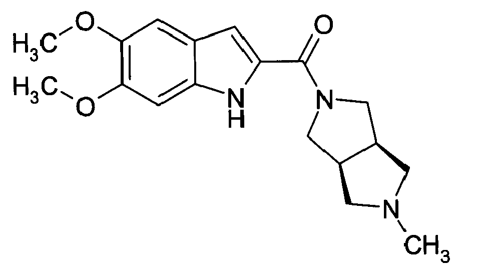

Prepared from 5,6-dimethoxy-1*H*-indole-2-carboxylic acid according to the method of example 1 to give the title compound as a colourless solid.
¹H NMR (400MHz, CD₃OD): δ 7.16 (1 H, s), 7.04 (1 H, s), 6.91 (1 H, s), 4.20-3.93 (2H, m), 3.92 (3H, s), 3.89 (3H, s), 3.86-3.77 (2H, m), 3.15-2.98 (2H, m), 2.83-2.79 (2H, m), 2.57-2.53 (2H, m), 2.38 (s, 3H) ppm.
MS (APCI) m/z 330 [M+H]⁺, 328 [M-H]⁻

### Example 24: 6,7-Dimethyl-2-{[(3aR,6aS)-5-methylhexahydropyrrolo[3,4-c]pyrrol-2(1H)-yl]carbonyl}-1H-benzimidazole

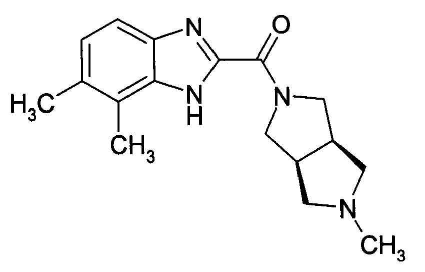

A solution of the trichlorobenzimidazole of preparation 9 (100mg, 0.37mmol) in a mixture of acetonitrile (3ml) and water (1ml) was treated with a solution of (3a*R*,6a*S*)-2-methyloctahydropyrrolo[3,4-*c*]pyrrole (95mg, 0.76mmol) in a mixture of acetonitrile (0.75ml) and water (0.25ml). The resulting solution was left to stir at room temperature for 2 hours. The solvent was removed *in vacuo* and the residue partitioned between dichloromethane (5ml) and saturated aqueous sodium bicarbonate (5ml). The organic phase was separated, dried (sodium sulfate) and the solvent removed *in vacuo*. Purification by flash column chromatography on silica gel eluting with dichloromethane : methanol : 880 ammonia (100:0:0 changing to 93:7:0.7, by volume) gave the title compound as a pale brown solid (48mg).
¹H NMR (400MHz, CD₃OD): δ 7.43-7.35 (1 H, m), 7.17 (1H, d), 4.43-4.27 (2H, m), 3.94-3.76 (2H, m), 3.15-2.98 (2H, m), 2.86-2.78 (2H, m), 2.56 (3H, s), 2.54-2.49 (2H, m), 2.43 (3H, s), 2.37 (3H, s) ppm.
MS (electrospray): m/z 299 [M+H]⁺, 297 [M-H]⁻

### Example 25: 6,7-Difluoro-2-{[(3aR,6aS)-5-methylhexahydropyrrolo[3,4-c]pyrrol-2(1H)-yl]carbonyl}-1H-benzimidazole

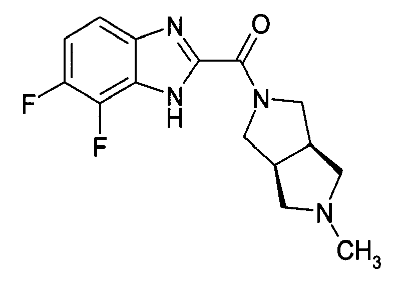

Prepared from the trichlorobenzimidazole of preparation 10 according to the method of example 24 with a reaction time of 2 hours to give the title compound as a pale brown solid.
¹H NMR (400MHz, CD₃OD): δ 7.43-7.39 (1H, m), 7.32-7.25 (1H, m), 4.45-4.33 (2H, m), 3.95-3.76 (2H, m), 3.15-2.98 (2H, m), 2.83-2.76 (2H, m), 2.58-2.53 (2H, m), 2.37 (3H, s) ppm.
MS (electrospray): m/z 307 [M+H]⁺, 305 [M-H]⁻

### Example 26: 7-Methyl-2-{[(3aR,6aS)-5-methylhexahydropyrrolo[3,4-c]pyrrol-2(1H)-yl]carbonyl}-1H-benzimidazole

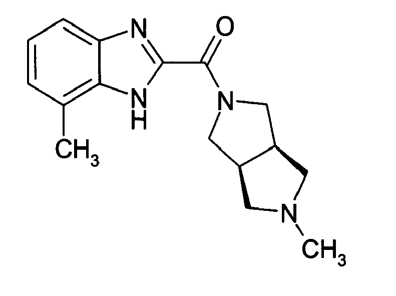

Prepared from the trichlorobenzimidazole of preparation 11 according to the method of example 24 with a reaction time of 2 hours to give the title compound as a pale brown solid.
¹H NMR (400MHz, CD₃OD): δ 7.45 (1H, d), 7.20 (1H, t), 7.09 (1H, d), 4.37-4.24 (2H, m), 3.90-3.71 (2H, m), 3.09-2.91 (2H, m), 2.79-2.71 (2H, m), 2.59 (3H, s), 2.49-2.44 (2H, m), 2.32 (3H, s) ppm.
MS (APCI): m/z 285 [M+H]⁺, 283 [M-H]⁻

### Example 27: 5,6-Difluoro-2-{[(3aR,6aS)-5-methylhexahydropvrrolo[3,4-c]pyrrol-2(1H)-yl]carbonyl}-1H-benzimidazole

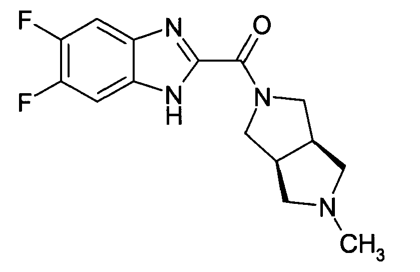

A solution of 5,6-difluoro-2-(trichloromethyl)-1*H*-benzimidazole (preparation 12) (500mg, 1.80mmol) in acetonitrile was treated with (3a*R*,6a*S*)-2-methyloctahydropyrrolo[3,4-c]pyrrole (255mg, 2.0mmol) and a solution of potassium carbonate (993mg, 7.2mmol). The resulting mixture was stirred at room temperature for 2 hours. Dichloromethane (30ml) and water (30ml) were added and the organic phase was separated, dried (sodium sulfate) and the solvent removed *in* vacuo. Purification by flash column chromatography on silica gel eluting with dichloromethane : methanol : 880 ammonia (98:2:0.2 changing to 90:10:1, by volume) gave the title compound as a pale brown solid (63mg).
¹H NMR (400MHz, CD₃OD): δ 7.37 (2H, t), 4.26-4.10 (2H, bm), 3.96-3.78 (2H, bm), 3.13-3.06 (2H, m), 2.68-2.59 (2H, m), 2.34 (3H, s) ppm.
MS (APCI): m/z 307 [M+H]⁺, 305 [M-H]⁻

### Example 28: 5,7-Difluoro-2-{[(3aR,6aS)-5-methylhexahydropyrrolo[3,4-c]pyrrol-2(1H)-yl]carbonyl}-1H-benzimidazole

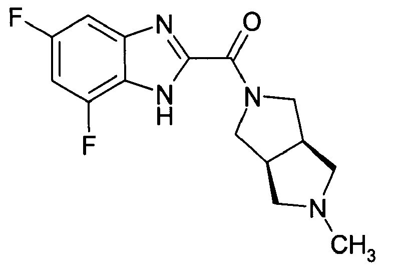

Prepared from the trichlorobenzimidazole of preparation 13 according to the method of example 24 with a reaction time of 2 hours to give the title compound as a pale brown solid.
¹H NMR (400MHz, CD₃OD): δ 7.15-7.12 (1H, m), 6.93-6.88 (1H, m), 4.40-4.29 (2H, m), 3.90-3.71 (2H, m), 3.12-2.94 (2H, m), 2.79-2.72 (2H, m), 2.53-2.49 (2H, m), 2.33 (3H, s) ppm.
MS (APCI): m/z 307 [M+H]⁺

### Example 29: 5-Chloro-7-methyl-2-{[(3aR,6aS)-5-methylhexahydropyrrolo[3,4-c]pyrrol-2(1H)-yl]carbonyl}-1H-benzimidazole

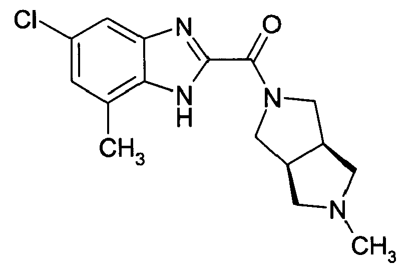

Prepared from the trichlorobenzimidazole of preparation 14 according to the method of example 24 with a reaction time of 2 hours to give the title compound as a pale brown solid.
¹H NMR (400MHz, CD₃OD): δ 7.44 (1H, s), 7.10 (1H, s), 4.39-4.27 (2H, m), 3.90-3.71 (2H, m), 3.10-2.94 (2H, m), 2.80-2.73 (2H, m), 2.58 (3H, m), 2.51-2.47 (m, 2H), 2.33 (3H, s) ppm.
MS (APCI): m/z 319/321 [M+H]⁺, 317/319 [M-H]⁻

### Example 30: 7-Chloro-5-methyl-2-{[(3aR,6aS)-5-methylhexahydropyrrolo[3,4-c]pyrrol-2(1H)-yl]carbonyl}-1H-benzimidazole

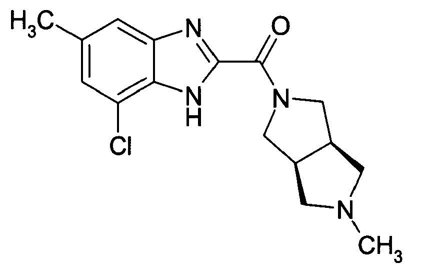

Prepared from the trichlorobenzimidazole of preparation 15 according to the method of example 24 with a reaction time of 16 hours to give the title compound as a pale orange solid.
¹H NMR (400MHz, CD₃OD): δ 7.31 (1 H, s), 7.19 (s, 1 H), 4.42-4.26 (2H, m), 3.92-3.71 (2H, m), 3.15-3.03 (2H, m), 2.82-2.72 (2H, m), 2.55-2.44 (2H, m), 2.35 (3H, s) ppm.
MS (APCI): m/z 319/321 [M+H]⁺, 317/319 [M-H]⁻

### Example 31: 5-Iodo-2-{[(3aR,6aS)-5-methylhexahydropyrrolo[3,4-c]pyrrol-2(1H)-yl]carbonyl}-1H-benzimidazole

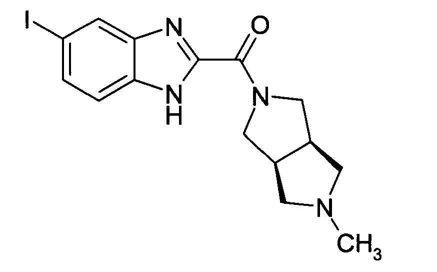

Prepared from the trichlorobenzimidazole of preparation 16 according to the method of example 24 with a reaction time of 3 hours to give the title compound as a pale brown solid.
¹H NMR (400MHz, CD₃OD): δ 8.07 (1 H, s), 7.65 (1 H, d), 7.51 (1 H, d), 4.42-4.31 (2H, m), 3.95-3.76 (2H, m), 3.17-2.98 (2H, m), 2.84-2.77 (2H, m), 2.58-2.54 (2H, m), 2.38 (3H, s) ppm.
MS (APCI): m/z 397 [M+H]⁺, 395 [M-H]⁻

### Example 32: Methyl 2-{[(3aR,6aS)-5-methylhexahydropyrrolo[3,4-c]pyrrol-2(1H)-yl]carbonyl}-1H-benzimidazole-5-carboxylate

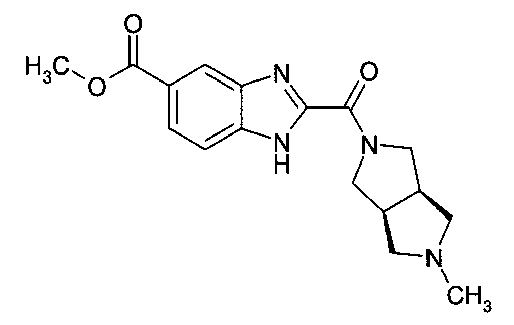

Prepared from the trichlorobenzimidazole of preparation 17 according to the method of example 24 with a reaction time of 3 hours to give the title compound as a pale brown solid.
¹H NMR (400MHz, CD₃OD): δ 8.41 (1 H, s), 8.03 (1 H, d), 7.73 (1 H, d), 4.44-4.34 (2H, m), 3.97 (3H, s), 3.96-3.77 (2H, m), 3.18-3.00 (2H, m), 2.87-2.80 (2H, m), 2.62-2.59 (2H, m), 2.37 (3H, s) ppm.
MS (APCI): m/z 329 [M+H]⁺, 327 [M-H]⁻

### Example 33: 5-Chloro-6-fluoro-2-{[(3aR,6aS)-5-methylhexahydropyrrolo [3,4-c]pyrrol-2(1H)-yl]carbonyl}-1H-benzimidazole

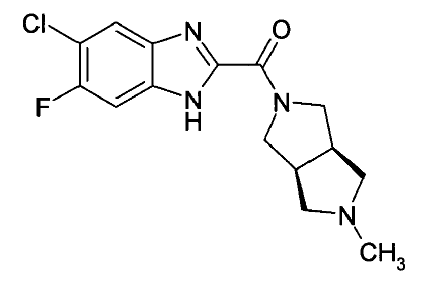

Prepared from the trichlorobenzimidazole of preparation 18 according to the method of example 24 with a reaction time of 1.5 hours to give the title compound as a colourless solid.
¹H NMR (400MHz, CD₃OD): δ 7.76 (1 H, d), 7.48 (1 H, d), 4.38-4.28 (2H, m), 3.90-3.71 (2H, m), 3.10-2.93 (2H, m), 2.78-2.71 (2H, m), 2.53-2.49 (2H, m), 2.33 (3H, s) ppm.
MS (APCI): m/z 323 [M+H]⁺, 321 [M-H]⁻

### Example 34: 5,6-Dimethyl-2-{[(3aR,6aS)-5-methylhexahydropyrrolo[3,4-c]pyrrol-2(1H)-yl]carbonyl}-1H-benzimidazole

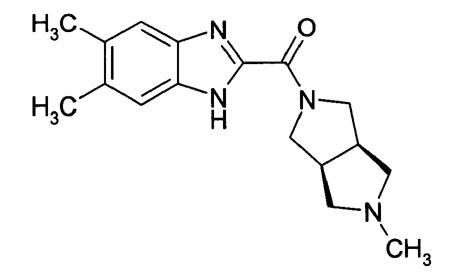

Prepared from the trichlorobenzimidazole of preparation 19 according to the method of example 24 with a reaction time of 1.5 hours to give the title compound as a pale yelow solid.
¹H NMR (400MHz, CD₃OD): δ 7.41 (2H, s), 4.34-4.21 (2H, m), 3.88-3.70 (2H, m), 3.08-2.92 (2H, m), 2.79-2.72 (2H, m), 2.49-2.45 (2H, m), 2.37 (6H, s), 2.32 (3H, s) ppm.
MS (APCI): m/z 297 [M+H]⁺, 299 [M-H]⁻

### Example 35: 5,6-Dichloro-2-{[(3aR,6aS)-5-methylhexahydropyrrolo[3,4-c]pyrrol-2(1H)-yl]carbonyl}-1H-benzimidazole

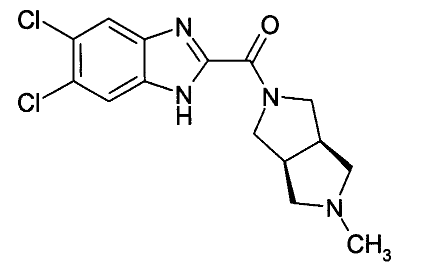

Prepared from the trichlorobenzimidazole of preparation 20 according to the method of example 24 with a reaction time of 16 hours to give the title compound as a pale brown solid.
¹H NMR (400MHz, CD₃OD): δ 7.81 (2H, s), 4.39-4.28 (2H, m), 3.92-3.72 (2H, m), 3.15-2.94 (2H, m), 2.79-2.70 (2H, m), 2.56-2.48 (2H, m), 2.35 (3H, s) ppm.
MS (APCI): m/z 339/341 [M+H]⁺, 337/339 [M-H]⁻

### Example 36: 2-{[(3aR,6aS)-5-methylhexahydropyrrolo[3,4-c]pyrrol-2(1H)-yl]carbonyl}-5-(trifluoromethyl)-1H-benzimidazole

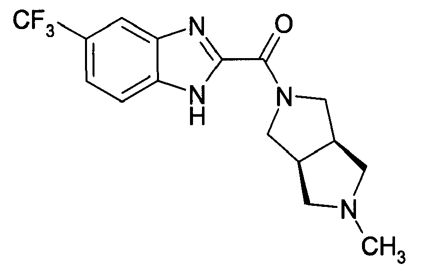

Prepared from the trichlorobenzimidazole of preparation 21 according to the method of example 27 with a reaction time of 16 hours to give the title compound as a colourless solid.
¹H NMR (400MHz, CD₃OD): δ 8.00 (1H, s), 7.80 (1 H, d), 7.59 (1 H, d), 4.42-4.30 (2H, m), 3.93-3.74 (2H, m), 3.14-2.95 (2H, m), 2.79-2.72 (2H, m), 2.55-2.51 (2H, m), 2.34 (3H, s) ppm.
MS (APCI): m/z 339 [M+H]⁺, 337 [M-H]⁻

### Example 37: 2-{[(3aR,6aS)-5-methylhexahydropyrrolo[3,4-c]pyrrol-2(1H)-yl]carbonyl}-1H-benzimidazole-5-carboxylic acid

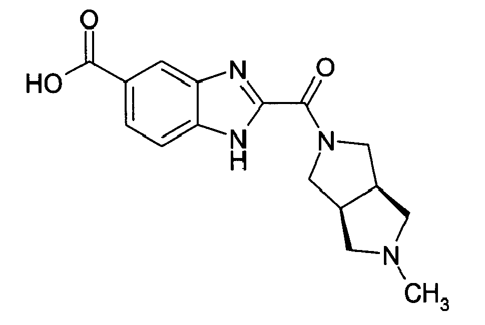

Methyl 2-{[(3a*R*,6a*S*)-5-methylhexahydropyrrolo[3,4-*c*]pyrrol-2(1*H*)-yl]carbonyl}-1*H*-benzimidazole-5-carboxylate (1.6g, 5.0mmol) in a mixture of water (8ml) and 1,4-dioxan (60ml) was treated with sodium hydroxide (0.6g, 15.0mmol) and the resulting suspension was heated to reflux for 16 hours. The reaction mixture was cooled to 10°C and neutralized with concentrated hydrochloric acid. The solvent was removed in vacuo and the residue solubilised in methanol, filtered and the solvent removed in vacuo to give the title compound as a colourless solid (1.41 g).
¹H NMR (400MHz, CD₃OD): δ 8.33 (1 H, bs), 7.98 (1H, d), 7.64-7.60 (1 H, m), 3.50-3.41 (2H, m), 3.20-3.12 (4H, m), 2.90-2.88 (2H, m), 2.68-2.60 (2H, m), 2.48 (3H, s) ppm.
MS (APCI): m/z 315 [M+H]⁺, 313 [M-H]⁻

### Example 38: 4-chloro-6-fluoro-2-{[(3aR,6aS)-5-methylhexahydropyrrolo [3,4-c]pyrrol-2(1H)-yl]carbonyl}-1H-benzimidazole

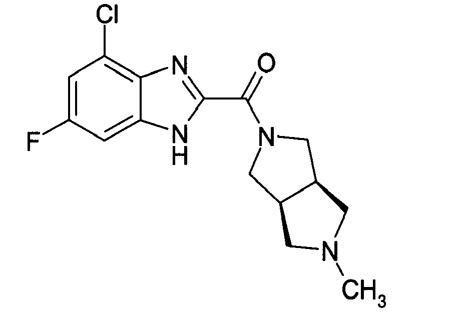

Prepared from the trichlorobenzimidazole of preparation 22 according to the method of example 24 with a reaction time of 16 hours to give the title compound as a pale yellow foam.
¹H NMR (400MHz, CD₃OD): δ 7.32-7.29 (1H, dd), 7.24-7.22 (1H, dd), 4.45-4.33 (2H, m), 3.94-3.75 (2H, m), 3.16-2.99 (2H, m), 2.84-2.77 (2H, m), 2.57-2.54 (2H, m), 2.37 (3H, s) ppm.
MS (APCI): m/z 323/325 [M+H]⁺, 321/323 [M-H]⁻

### Example 39: 4,6-dichloro-2-{[(3aR,6aS)-5-methylhexahydropyrrolo[3,4-c]pyrrol-2(1H)-yl]carbonyl}-1H-benzimidazole

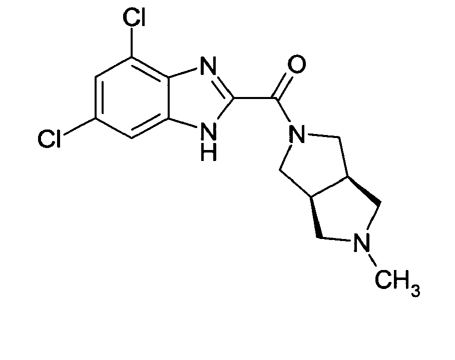

Prepared from the trichlorobenzimidazole of preparation 23 according to the method of example 24 with a reaction time of 16 hours to give the title compound as a pale yellow foam.
¹H NMR (400MHz, CD₃OD): δ 7.60 (1 H, s), 7.38 (1 H, s), 4.44-4.32 (2H, m), 3.95-3.75 (2H, m), 3.17-2.99 (2H, m), 2.84-2.77 (2H, m), 2.58-2.54 (2H, m), 2.37 (3H, s) ppm.
MS (APCI): m/z 339/341 [M+H]⁺, 337/339 [M-H]⁻

### Example 40: 2-{[(3aR,6aS)-5-methylhexahydropyrrolo[3,4-c]pyrrol-2(1H)-yl]carbonyl}-5-phenyl-1H-benzimidazole

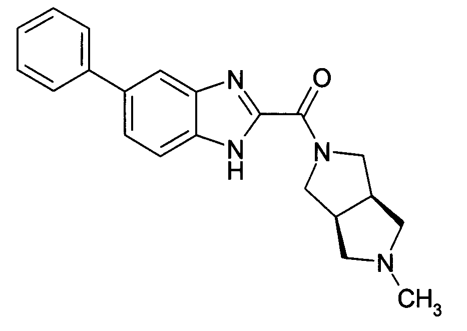

Prepared from the trichlorobenzimidazole of preparation 24 according to the method of example 24 with a reaction time of 16 hours to give the title compound as a colourless solid.
¹H NMR (400MHz, CD₃OD): δ 7.88 (1 H, s), 7.75 (1H, d), 7.69-7.67 (2H, m), 7.63-7.61 (1 H, m), 7.49-7.45 (2H, m), 7.38-7.34 (1 H, m), 4.43-4.31 (2H, m), 3.94-3.76 (2H, m), 3.13-2.97 (2H, m), 2.82-2.75 (2H, m), 2.55-2.51 (2H, m), 2.36 (3H, s) ppm.
MS (APCI): m/z 347 [M+H]⁺, 345 [M-H]⁻

### Example 41:5-fluoro-4-methyl-2-{[(3aR,6aS)-5-methylhexahvdropyrrolo [3,4-c]pyrrol-2(1H)-yl]carbonyl}-1H-benzimidazole

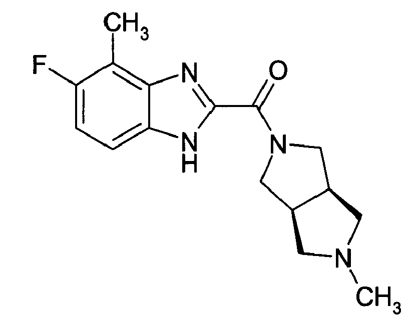

Prepared from the trichlorobenzimidazole of preparation 25 according to the method of example 24 with a reaction time of 16 hours to give the title compound as a yellow foam.
¹H NMR (400MHz, CD₃OD): δ 7.49-7.46 (1H, m), 7.13-7.08 (1H, m), 4.43-4.31 (2H, m), 3.95-3.76 (2H, m), 3.15-2.98 (2H, m), 2.85-2.77 (2H, m), 2.53 (3H, s), 2.37 (3H, s) ppm.
MS (APCI): m/z 303 [M+H]⁺, 301 [M-H]⁻

### Example 42: 5-Chloro-2-{[(3aR,6aS)-5-ethylhexahydropyrrolo[3,4-c]pyrrol-2(1H)-yl]carbonyl}-1H-indole

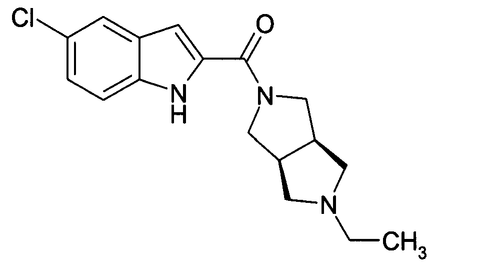

A solution of the amine of example 8 (170mg, 0.58mmol) in dichloromethane (5ml) was treated with acetaldehyde (66µl, 1.18mmol), acetic acid (33µl, 0.58mmol) and sodium triacetoxy borohydride (147mg, 70mmol) and the resulting mixture left to stir at room temperature for 16 hours. Further dichloromethane (20ml) and sat. aq. sodium hydrogen carbonate (20ml) was added and the organic phase separated. The aqueous phase was extracted with further dichloromethane (20ml) and the combined organics dried (sodium sulphate) and the solvent removed in vacuo. The residue was purified by flash column chromatography on silica gel eluting with dichloromethane : methanol : 880 ammonia (99:1:0.1 changing to 90:10:1, by volume) to give the title compound as a pale yellow solid (60mg).
¹H NMR (400MHz, CD₃OD): δ 7.67 (1 H, s), 7.46 (1 H, d), 7.23 (1 H, dd), 6.97 (1 H, d), 4.19-3.74 (4H, bm), 3.19-2.97 (2H, m), 2.92-2.85 (2H, m), 2.58-2.51 (4H, m), 1.16 (3H, t) ppm.
MS (APCI): m/z 318/320 [M+H]⁺, 316/318 [M-H]⁻

### Example 43: 5-Chloro-2-{[(3aR,6aS)-5-isopropylhexahydropyrrolo[3,4-c]pyrrol-2(1H)-yl]carbonyl}-1H-indole

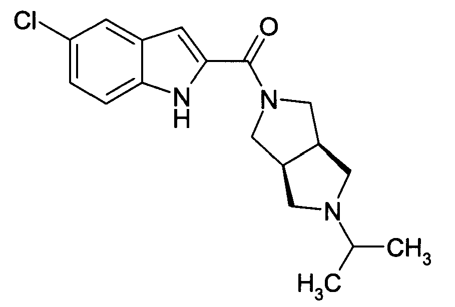

Prepared according to the method for example 42 using the amine of example 8 and acetone to give the title compound as a colourless solid.
¹H NMR (400MHz, DMSO_{d6}): δ 11.75 (1 H, s), 7.69 (1 H, s), 7.46 (1 H, dd), 7.21 (1H, dd), 6.93 (1H, dd), 4.16-4.01 (1H, m), 3.94-3.84 (1H, m), 3.73-3.64 (1 H, m), 3.59-3.50 (1 H, m), 2.98-2.77 (2H, m), 2.63-2.53 (4H, m), 2.34 (1 H, m), 1.02 (6H, d) ppm.
MS (APCI): m/z 332/334 [M+H]⁺, 330/332 [M-H]⁻

### Example 44: 5-Chloro-2-{[(3aR,6aS)-5-ethylhexahydroxyrrolo[3,4-c]pyrrol-2(1H)-yl]carbonyl}-1H-benzimidazole

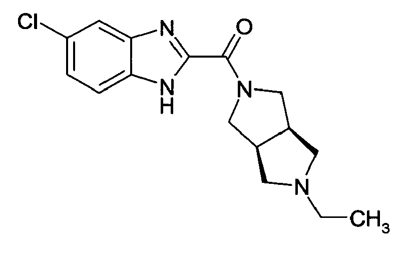

Prepared according to the method for example 42 using the amine of example 6 and acetaldehyde to give the title compound as a colourless solid.
¹H NMR (400MHz, CD₃OD): δ 7.70-7.68 (1H, m), 7.66 (1H, s), 7.30 (1H, d), 4.42-4.33 (2H, m), 3.93-3.79 (2H, m), 3.13-2.90 (4H, m), 2.61-2.53 (4H, m), 1.17 (3H, t) ppm.
MS (APCI): m/z 319/321 [M+H]⁺, 317/319 [M-H]⁻

### Example 45: 5-Chloro-2-{[(3aR,6aS)-5-isopropylhexahydropyrrolo[3,4-c]pyrrol-2(1H-yl]carbonyl}-1H-benzimidazole

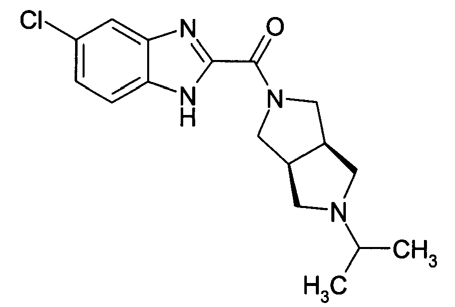

Prepared according to the method for example 42 using the amine of example 6 and acetone to give the title compound as a colourless solid.
¹H NMR (400MHz, CD₃OD): δ 7.70-7.67 (2H, m), 7.36-7.34 (1 H, m), 4.43-4.31 (2H, m), 3.92-3.80 (2H, m), 3.13-2.98 (4H, m), 2.56-2.46 (3H, m), 1.17 (6H, d) ppm.
MS (APCI): m/z 333/335 [M+H]⁺, 331/333 [M-H]⁻

### Example 46: 2-[(3aR,6aS)-5-[(5-Chloro-1H-benzimidazol-2-yl)carbonyl]hexahydropyrrolo[3,4-c]pyrrol-2(1H)-yl]ethanol

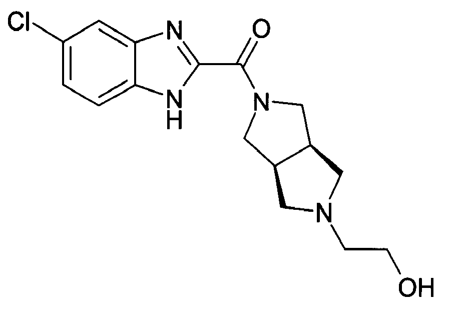

Prepared according to the method for example 42 using the amine of example 6 and 1,4-dioxane-2,5-diol to give the title compound as a colourless solid.
¹H NMR (400MHz, CD₃OD): δ 7.70-7.65 (2H, m), 7.36-7.33 (1H, m), 4.38-4.36 (2H, m), 3.94-3.78 (2H, m), 3.70 (2H, t), 3.13-2.92 (4H, m), 2.73-2.65 (4H, m).
MS (APCI): m/z 335/337 [M+H]⁺, 333/335 [M-H]⁻

### BIOLOGY SECTION

### H₄ Binding

Cell pellets from CHO cells expressing the histamine H₄ receptor were homogenised in ice-cold 50mM Tris-HCl/0.5mM CaCl₂ buffer containing a protease inhibitor cocktail (Roche®, United Kingdom) using a ground glass homogeniser. Homogenates were centrifuged at 48000g for 30min at 4°C.
The membrane pellet was resuspended in fresh buffer and the centrifugation step was repeated as described above. The membrane pellet was resuspended in 50mM Tris-HCl in the same volume as the original cell pellet. Aliquots of membrane preparations were stored at -80°C and were used for [³H]-Histamine binding experiments.

Cell membranes (20-35µg/well) were incubated for 90min shaking at room temperature with 3nM [³H]-Histamine (23Ci/mmol) in 50mM Tris-HCl (pH 7.4), with or without competing H₄ ligands. The reaction was terminated by rapid filtration through 0.5% polyethylenimine-soaked Unifilter GF/B plates (Packard) followed by three washes with 1ml ice-cold 50mM Tris-HCl. Filters were dried for 45min at 45°C and bound radiolabel was determined using scintillation counting techniques. Non-specific binding was defined with 5µM clobenpropit. For competition binding studies, Ki values were calculated from the IC₅₀ value based on an experimentally determined ligand K_{d} of 3.5nM and a ligand concentration of 3nM according to the Cheng-Prussoff equation where; Kᵢ = (IC₅₀)/(1 +([L]/K_{d}))

The compounds of the Examples have been tested in the H₄ assay described above and were found to have a Kᵢ value of less than 10 µM in the H₄ cell based functional assay. Preferred examples have a Kᵢ value of less than 1 µM in the H₄ cell based functional assay. Most preferred examples have a Kᵢ value of less than 100 nM in the H₄ cell based functional assay.

## Claims

1. A compound of formula (I) 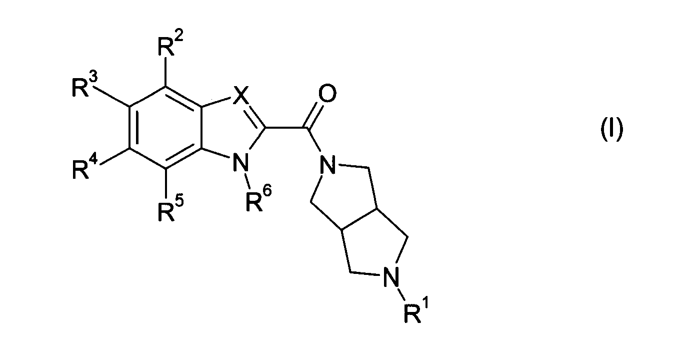 or pharmaceutically acceptable salts and solvates thereof, wherein:
• R¹ is H or (C₁-C₄) alkyl optionally substituted with a hydroxy;
• X is N or C-R⁹ wherein R⁹ is H or methyl;
• R⁶ is H or methyl; and
• R², R³, R⁴ and R⁵ are independently selected from H, halo, cyano, (C₁-C₄)alkyl, (C₁-C₄)alkoxy, trifluoromethyl, trifluoromethoxy, hydroxy, (CH₂)ₙ-C(O)O-R⁷, (CH₂)ₙ-O-(CH₂)ₘ-R⁸ and (CH₂)ₙ-R⁸,
wherein n and m are both independently 0 or 1, R⁷ is H or (C₁₋C₄)alkyl and R⁸ is phenyl.

2. A compound of formula (I) according to claim 1 wherein R¹ is H or methyl.

3. A compound of formula (I) according to claim 1 or 2 wherein R², R³, R⁴ and R⁵ are independently selected from H, halo, methyl, hydroxy, phenyl, and COOH.

4. A compound of formula (I) according to claim 3 wherein R², R³, R⁴ and R⁵ are independently selected from H, fluoro, chloro, bromo, hydroxy and methyl.

5. A compound of formula (I) according to any one of claims 1 to 4
wherein R⁶ is H.

6. A compound of formula (I) according to any one of claims 1 to 5
wherein X is N or CH.

7. A compound of formula (I) according to any one of claims 1 to 6
wherein at least two of R², R³, R⁴ and R⁵ are H.

8. A compound of formula (I) according to any one of claims 1 to 7 which has the Cis configuration (3a*R*,6a*S*).

9. A compound of formula (I) according to claim 1, which is selected from:
2-[(5-Methylhexahydropyrrolo[3,4-c]pyrrol-2(1H)-yl)carbonyl]-1H-benzimidazole;
5-Methoxy-2-{[(3a*R*,6a*S*)-5-methylhexahydropyrrolo[3,4-*c*]pyrrol-2(1*H*)-yl]carbonyl}-1*H*-benzimidazole;
5-Fluoro-2-{[(3a*R*,6a*S*)-5-methylhexahydropyrrolo[3,4-*c*]pyrrol-2(1*H*)-yl]carbonyl}-1*H*-benzimidazole;
5-Methyl-2-{[(3a*R*,6a*S*)-5-methylhexahydropyrrolo[3,4-*c*]pyrrol-2(1*H*)-yl]carbonyl}-1*H*-benzimidazole;
5-Chloro-2-{[(3a*R*,6a*S*)-5-methylhexahydropyrrolo[3,4-*c*]pyrrol-2(1*H*)-yl]carbonyl}-1*H*-benzimidazole;
5-Chloro-2-[(3a*R*,6a*S*)-hexahydropyrrolo[3,4-*c*]pyrrol-2(1*H*)-ylcarbonyl]-1*H-*benzimidazole;
5-Chloro-2-{[(3a*R*,6a*S*)-5-methylhexahydropyrrolo[3,4-*c*]pyrrol-2(1*H*)-yl]carbonyl}-1*H*-indole;
5-Chloro-2-{[(3a*R*,6a*S*)-hexahydropyrrolo[3,4-*c*]pyrrol-2(1*H*)-yl]carbonyl}-1*H*-indole;
5-Methoxy-3-methyl-2-{[(3a*R*,6a*S*)-5-methylhexahydropyrrolo[3,4-*c*]pyrrol-2(1 *H*)-yl]carbonyl}-1*H*-indole;
5-(Benzyloxy)-2-{[(3a*R*,6a*S*)-5-methylhexahydropyrrolo[3,4-*c*]pyrrol-2(1*H*)-yl]carbonyl}-1*H*-indole;
6-Methyl-2-{[(3a*R*,6a*S*)-5-methylhexahydropyrrolo[3,4-*c*]pyrrol-2(1*H*)-yl]carbonyl}-1*H*-indole;
4-Methoxy-2-{[(3a*R*,6a*S*)-5-methylhexahydropyrrolo[3,4-*c*]pyrrol-2(1*H*)-yl]carbonyl}-1*H*-indole;
6-Chloro-2-{[(3a*R*,6a*S*)-5-methylhexahydropyrrolo[3,4-*c*]pyrrol-2(1*H*)-yl]carbonyl}-1*H*-indole;
5-Methoxy-2-{[(3a*R*,6a*S*)-5-methylhexahydropyrrolo[3,4-*c*]pyrrol-2(1*H*)-yl]carbonyl}-1*H*-indole;
2-{[(3a*R*,6a*S*)-5-Methylhexahydropyrrolo[3,4-*c*]pyrrol-2(1*H*)-yl]carbonyl}-1*H-*indol-5-ol;
5-Fluoro-2-{[(3a*R*,6a*S*)-5-methylhexahydropyrrolo[3,4-*c*]pyrrol-2(1*H*)-yl]carbonyl}-1 *H*-indole;
5-Methyl-2-{[(3a*R*,6a*S*)-5-methylhexahydropyrrolo[3,4-*c*]pyrrol-2(1*H*)-yl]carbonyl}-1 *H*-indole;
3,5-Dimethyl-2-{[(3a*R*,6a*S*)-5-methylhexahydropyrrolo[3,4-*c*]pyrrol-2(1*H*)-yl]carbonyl}-1*H*-indole;
5-Chloro-3-methyl-2-{[(3a*R*,6a*S*)-5-methylhexahydro-pyrrolo[3,4-*c*]pyrrol-2(1*H*)-yl]carbonyl}-1*H*-indole;
1-Methyl-2-{[(3a*R*,6a*S*)-5-methylhexahydropyrrolo[3,4-c]pyrrol-2(1*H*)-yl]carbonyl}-1*H*-indole;
5-Bromo-2-{[(3a*R*,6a*S*)-5-methylhexahydropyrrolo[3,4-*c*]pyrrol-2(1*H*)-yl]carbonyl}-1*H*-indole;
7-Fluoro-2-{[(3a*R*,6a*S*)-5-methylhexahydropyrrolo[3,4-*c*]pyrrol-2(1*H*)-yl]carbonyl}-1*H*-indole;
5,6-Dimethoxy-2-{[(3a*R*,6a*S*)-5-methylhexahydropyrrolo[3,4-*c*]pyrrol-2(1*H*)-yl]carbonyl}-1*H*-indole;
6,7-Dimethyl-2-{[(3a*R*,6a*S*)-5-methylhexahydropyrrolo[3,4-*c*]pyrrol-2(1*H*)-yl]carbonyl}-1*H*-benzimidazole;
6,7-Difluoro-2-{[(3a*R*,6a*S*)-5-methylhexahydropyrrolo[3,4-*c*]pyrrol-2(1*H*)-yl]carbonyl}-1*H*-benzimidazole;
7-Methyl-2-{[(3a*R*,6a*S*)-5-methylhexahydropyrrolo[3,4-*c*]pyrrol-2(1*H*)-yl]carbonyl}-1*H*-benzimidazole;
5,6-Difluoro-2-{[(3a*R*,6a*S*)-5-methylhexahydropyrrolo[3,4-*c*]pyrrol-2(1*H*)-yl]carbonyl}-1*H*-benzimidazole;
5,7-Difluoro-2-{[(3a*R*,6a*S*)-5-methylhexahydropyrrolo[3,4-*c*]pyrrol-2(1*H*)-yl]carbonyl}-1H-benzimidazole;
5-Chloro-7-methyl-2-{[(3a*R*,6a*S*)-5-methylhexahydropyrrolo[3,4-*c*]pyrrol-2(1 *H*)-yl]carbonyl}-1*H*-benzimidazole;
7-Chloro-5-methyl-2-{[(3a*R*,6a*S*)-5-methylhexahydropyrrolo[3,4-*c*]pyrrol-2(1*H*)-yl]carbonyl}-1H-benzimidazole;
5-Iodo-2-{[(3a*R*,6a*S*)-5-methylhexahydropyrrolo[3,4-c]pyrrol-2(1*H*)-yl]carbonyl}-1*H*-benzimidazole;
Methyl 2-{[(3a*R*,6a*S*)-5-methylhexahydropyrrolo[3,4-*c*]pyrrol-2(1*H*)-yl]carbonyl}-1*H*-benzimidazole-5-carboxylate;
5-Chloro-6-fluoro-2-{[(3a*R*,6a*S*)-5-methylhexahydropyrrolo [3,4-c]pyrrol-2(1 *H*)-yl]carbonyl}-1*H*-benzimidazole;
5,6-Dimethyl-2-{[(3a*R*,6a*S*)-5-methylhexahydropyrrolo[3,4-*c*]pyrrol-2(1*H*)-yl]carbonyl}-1*H*-benzimidazole;
5,6-Dichloro-2-{[(3a*R*,6a*S*)-5-methylhexahydropyrrolo[3,4-*c*]pyrrol-2(1*H*)-yl]carbonyl}-1*H*-benzimidazole;
2-{[(3a*R*,6a*S*)-5-Methylhexahydropyrrolo[3,4-*c*]pyrrol-2(1*H*)-yl]carbonyl}-5-(trifluoromethyl)-1 *H*-benzimidazole;
2-{[(3a*R*,6a*S*)-5-Methylhexahydropyrrolo[3,4-*c*]pyrrol-2(1*H*)-yl]carbonyl}-1*H-*benzimidazole-5-carboxylic acid;
4-Chloro-6-fluoro-2-{[(3a*R*,6a*S*)-5-methylhexahydropyrrolo [3,4-*c*]pyrrol-2(1 *H*)-yl]carbonyl}-1*H*-benzimidazole;
4,6-Dichloro-2-{[(3a*R*,6a*S*)-5-methylhexahydropyrrolo[3,4-*c*]pyrrol-2(1*H*)-yl]carbonyl}-1*H*-benzimidazole;
2-{[(3a*R*,6a*S*)-5-Methylhexahydropyrrolo[3,4-*c*]pyrrol-2(1*H*)-yl]carbonyl}-5-phenyl-1 *H*-benzimidazole;
5-Fluoro-4-methyl-2-{[(3a*R*,6a*S*)-5-methylhexahydropyrrolo [3,4-c]pyrrol-2(1*H*)-yl]carbonyl}-1*H*-benzimidazole;
5-Chloro-2-{[(3a*R*,6a*S*)-5-ethylhexahydropyrrolo[3,4-*c*]pyrrol-2(1*H*)-yl]carbonyl}-1*H*-indole;
5-Chloro-2-{[(3a*R*,6a*S*)-5-isopropylhexahydropyrrolo[3,4-*c*]pyrrol-2(1*H*)-yl]carbonyl}-1*H*-indole;
5-Chloro-2-{[(3a*R*,6a*S*)-5-ethylhexahydropyrrolo[3,4-*c*]pyrrol-2(1*H*)-yl]carbonyl}-1*H*-benzimidazole;
5-Chloro-2-{[(3a*R*,6a*S*)-5-isopropylhexahydropyrrolo[3,4-*c*]pyrrol-2(1*H*)-yl]carbonyl}-1*H*-benzimidazole and
2-[(3a*R*,6a*S*)-5-[(5-Chloro-1 *H*-benzimidazol-2-yl)carbonyl]-hexahydropyrrolo-[3,4-*c*]pyrrol-2(1*H*)-yl]ethanol.

10. A pharmaceutical composition including a compound of the formula (I) or a pharmaceutically acceptable salt or solvate thereof, as defined in any one of claims 1 to 9, together with a pharmaceutically acceptable excipient.

11. A compound of the formula (I) as defined in any one of claims 1 to 9 or a pharmaceutically acceptable salt or solvate thereof, for use as a medicament.

12. The use of a compound of the formula (I) as defined in any one of claims 1 to 9 or a pharmaceutically acceptable salt or solvate thereof, for the manufacture of a medicament to treat a disease for which a H₄ ligand is indicated.

13. The use of a compound of the formula (I) according to claim 12, for the manufacture of a medicament to treat a disease selected from inflammatory diseases, respiratory diseases (e.g. adult respiratory distress syndrome, acute respiratory distress syndrome, bronchitis, chronic bronchitis, chronic obstructive pulmonary disease, cystic fibrosis, asthma, emphysema, rhinitis, chronic sinusitis), allergy; allergy-induced airway responses, allergic rhinitis, viral rhinitis, non-allergic rhinitis, perennial and seasonal rhinitis, nasal congestion, allergic congestion, female and male sexual dysfunction, skin diseases such as dermatitis and psoriasis, cardiac dysfunctions such as myocardial ischaemia and arrythmia, diseases of the gastrointestinal tract such as inflammatory bowel disease, Crohn's disease and colitis ulcerosa, cancer, rheumatoid arthritis, hypotension, pain and overactive bladder conditions.

14. A method of treatment of a mammal, including a human being, suffering from a disease for which a H₄ ligand is indicated, comprising administering to said mammal an effective amount of a compound of the formula (I) as defined in any one of claims 1 to 9 or a pharmaceutically acceptable salt, solvate or composition thereof.

15. A method according to claim 14 wherein the disease is selected from those of claim 13.

16. A process comprising the step of coupling an acid of formula (II) : 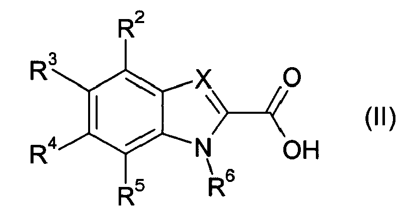 with an amine of formula (III): 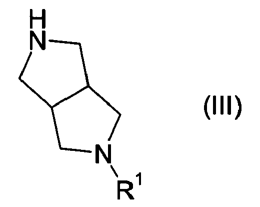 wherein R¹, R², R³, R⁴, R⁵, R⁶ and X are as defined in any one of claims 1 to 5, to obtain a compound of formula (I) as defined in any one of claims 1 to 5.

17. A combination of a compound of formula (I) as defined in any one of claims 1 to 9 and another pharmacologically active agent selected from:
• Histamine H₁ receptor antagonists, for instance loratidine, desloratidine, fexofenadine and cetirizine
• Histamine H₃ receptor antagonists
• Histamine H₂ receptor antagonists
• Leukotriene antagonists, including antagonists of LTB₄, LTC₄, LTD₄, and LTE₄, in particular Montelukast
• Phosphodiesterase PDE4 inhibitors
• neurotransmitter re-uptake inhibitors, for instance fluoxetine, setraline, paroxetine, ziprasidone
• 5-Lipoxygenase (5-LO) inhibitors or 5-lipoxygenase activating protein (FLAP) antagonists,
• α₁- and α₂-adrenoceptor agonist vasoconstrictor sympathomimetic agents for decongestant use,
• Muscarinic M3 receptor antagonists or anticholinergic agents,
• β₂-adrenoceptor agonists,
• Theophylline,
• Sodium cromoglycate,
• COX-1 inhibitors (NSAIDs) and COX-2 selective inhibitors,
• Oral or inhaled Glucocorticosteroids,
• Monoclonal antibodies active against endogenous inflammatory entities,
• Anti-tumor necrosis factor (anti-TNF-α) agents,
• Adhesion molecule inhibitors including VLA-4 antagonists,
• Kinin-B₁ - and B₂-receptor antagonists,
• Immunosuppressive agents,
• Inhibitors of matrix metalloproteases (MMPs),
• Tachykinin NK₁, NK₂ and NK₃ receptor antagonists,
• Elastase inhibitors,
• Adenosine A2a receptor agonists,
• Inhibitors of urokinase,
• Compounds that act on dopamine receptors, e.g. D2 agonists,
• Modulators of the NFκβ pathway, e.g. IKK inhibitors,
• Agents that can be classed as mucolytics or anti-tussive,
• antibiotics,
• p38 MAP kinase inhibitors
• Phosphodiesterase PDE5 inhibitors
• Prostaglandin D2 receptor antagonists (DP1 and CRTH2)
• Inhibition of Prostaglandin D synthase (PGDS)
• Inhibitors of phosphoinositide-3-kinase, and
• Inhibitors of Syk tyrosine kinase.
